(19) Europäisches Patentamt
European Patent Office
Office européen des brevets

(11) EP 2 041 153 B1

(12) FASCICULE DE BREVET EUROPEEN

(45) Date de publication et mention de la délivrance du brevet:
**21.10.2009 Bulletin 2009/43**

(21) Numéro de dépôt: **07823569.4**

(22) Date de dépôt: **12.07.2007**

(51) Int Cl.:
***C07H 17/00*** *(2006.01)* ***C07H 1/00*** *(2006.01)*
***A61K 31/7032*** *(2006.01)*

(86) Numéro de dépôt international:
**PCT/FR2007/051647**

(87) Numéro de publication internationale:
**WO 2008/007027 (17.01.2008 Gazette 2008/03)**

# (54) NOUVEAUX DERIVES DE 5-THIOXYLOPYRANOSE

NEUE 5-THIOXYLPYRANOSE-DERIVATE

NEW 5-THIOXYLOPYRANOSE DERIVATIVES

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **13.07.2006 FR 0652954**

(43) Date de publication de la demande:
**01.04.2009 Bulletin 2009/14**

(73) Titulaire: **LABORATOIRES FOURNIER SA**
**21000 Dijon (FR)**

(72) Inventeurs:
- **THOMAS, Didier**
  **F-21850 Saint-apollinaire (FR)**
- **BONDOUX, Michel**
  **F-21121 Fontaine-les-dijon (FR)**
- **BARBEROUSSE, Véronique**
  **F-21121 Hauteville Les Dijon (FR)**
- **PEYROU, Vincent**
  **F-21121 Hauteville Les Dijon (FR)**

(74) Mandataire: **Hubert, Philippe et al**
**Cabinet Beau de Loménie**
**158, rue de l'Université**
**75340 Paris Cédex 07 (FR)**

(56) Documents cités:
**EP-A1- 0 421 829** **EP-A2- 0 365 397**
**FR-A1- 2 860 234**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 041 153 B1

Printed by Jouve, 75001 PARIS (FR)

## Description

La présente invention concerne de nouveaux composés du 5-thioxylose, préférentiellement des dérivés de type 5-thioxylopyranose, ainsi que leur procédé de préparation et leur utilisation en tant que principe actif de médicaments, notamment destinés au traitement ou à la prévention des thromboses.

### *Art antérieur*

On connaît déjà des dérivés du D-xylose, par exemple par les documents EP 051 023 B1, US 4 877 808, EP 421 829 B1, WO 05/030 785 ou par la publication J. Med. Chem. Vol. 36 n° 7, p 898-903. Ces composés connus sont préconisés pour réduire les risques de thrombose veineuse chez l'homme. Le mécanisme d'action de ces composés semble être un effet sur les glycosaminoglycanes plasmatiques (J. Biol. Chem., Vol 270 n° 6 p 2662-68, Thromb. Haemost. 1999, 81 p 945-950). EP 0 365 397 et FR 2 860 234 décrivent également des dérivés anti-thrombotiques du D-xylose.

### *Objet de l'invention*

On a maintenant découvert une nouvelle famille de composés, dérivés du thioxylose, qui présentent une bonne activité antithrombotique et qui peuvent être synthétisés de manière efficace.

### *Description*

Les nouveaux composés selon l'invention sont caractérisés en ce qu'ils sont choisis parmi :

a) les composés de formule :

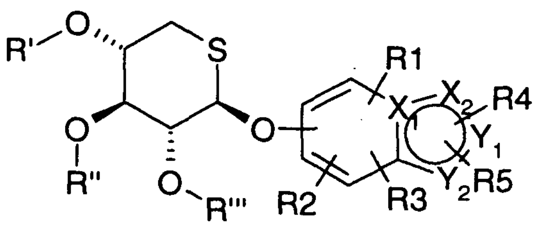

(I)

dans laquelle :

- le groupe pentapyranosyle représente un groupe 5-thio-β-D-xylopyranosyle libre ou substitué,
- R', R" et R"' représentent chacun indépendamment un atome d'hydrogène, un groupe acyle en $C_2$-$C_6$, ou deux contigus d'entre eux forment un pont 1-méthyléthylidène,
- $X_1$ et $X_2$ représentent chacun un atome de carbone ou d'azote,
- $Y_1$ et $Y_2$ représentent chacun indépendamment l'un de l'autre un atome de carbone, d'azote, de soufre ou d'oxygène, avec la condition que si $Y_2$ représente un atome d'oxygène ou de soufre, $Y_1$ représente un atome de carbone ou d'azote.
- $R_1$, $R_2$, $R_3$, $R_4$ et $R_5$ représentent, indépendamment l'un de l'autre, un atome d'hydrogène, un groupe -$COOR_6$ où $R_6$ représente un atome d'hydrogène ou un alkyle en $C_1$-$C_4$, un groupe alkyle en $C_1$-$C_4$ éventuellement substitué par un noyau phényle, un atome d'halogène ou un groupe -$COOR_6$, un groupe alcoxy en $C_1$-$C_4$, un groupe acyle en $C_2$-$C_6$, un groupe benzoyle ou un noyau phényle ;

b) les sels d'addition des composés de formule (I) ;

L'invention concerne également les composés de formule I pour leur utilisation en tant que substance pharmacologiquement active.

En particulier, l'invention concerne l'utilisation d'au moins une substance choisie parmi les composés de formule I et leurs sels d'addition non toxiques pour la préparation d'un médicament, utile en thérapeutique humaine ou animale, destiné à la prévention ou au traitement des thromboses, notamment les thromboses veineuses. Les composés selon l'invention étant actifs selon un mode d'action faisant intervenir les glycosaminoglycanes, ils pourront être utiles en tant que principe actif d'un médicament destiné au traitement ou à la prévention de toute autre maladie dans laquelle les

glycosaminoglycanes sont impliqués.

### *Description détaillée*

Dans la formule I, on entend par groupe alkyle en $C_1$-$C_4$ une chaîne hydrocarbonée saturée linéaire ou ramifiée ayant de 1 à 4 atomes de carbone ou partiellement ou totalement cyclisée, la portion cyclisée ayant 3 ou 4 atomes de carbone. Des exemples de groupes alkyle en $C_1$-$C_4$ sont notamment les groupes méthyle, éthyle, propyle, butyle, 1-méthyléthyle, 1,1-diméthyléthyle, 1-méthylpropyle, 2-méthylpropyle, cyclopropyle ou cyclo-propylméthyle.

Par groupe acyle en $C_2$-$C_6$, on entend un groupe R-CO-, dans lequel R représente un groupe alkyle tel que défini précédemment ayant de 1 à 5 atomes de carbone. Des exemples de groupes acétyles en $C_2$-$C_6$ sont notamment les groupes acétyle, propanoyle, butanoyle, pentanoyle, hexanoyle, ainsi que leurs homologues dans lesquels la chaîne peut être ramifiée.

Par groupe alcoxy en $C_1$-$C_4$ on entend un groupe RO-, dans lequel R présente un groupe alkyle ayant de 1 à 4 atomes de carbone tel que défini précédemment. A titre d'exemples de groupes alcoxy en $C_1$-$C_4$ on peut citer les groupes méthoxy, éthoxy, propoxy, butoxy, 1-méthyléthoxy, 1,1-diméthyléthoxy, 1-méthylpropoxy, 2-méthylpropoxy ou cyclopropylméthoxy.

Par sels d'addition, on entend les sels d'addition obtenus par réaction d'un composé de formule I avec un acide minéral ou organique. De préférence, il s'agit des sels d'addition pharmaceutiquement acceptables. Les hydrates ou solvates des composés de formule I ou des sels des composés de formule I font également partie intégrante de l'invention.

Parmi les acides minéraux convenant pour salifier un composé basique de formule I, on préfère les acides chlorhydrique, bromhydrique, phosphorique et sulfurique. Parmi les acides organiques convenant pour salifier un composé basique de formule I, on préfère les acides méthanesulfonique, benzènesulfonique, toluènesulfonique, maléïque, fumarique, oxalique, citrique, tartrique, lactique et trifluoroacétique.

Des composés illustratifs de l'invention sont ceux de formule 1 dans laquelle :

- le groupe pentapyranosyle représente un groupe 5-thio-β-D-xylopyranosyle libre ou acylé, de préférence acétylé ; ou
- $X_1$ et $Y_2$ représentent un atome d'azote et $X_2$ et $Y_1$ représentent un atome de carbone ; ou
- $Y_2$ représente un atome d'oxygène et $X_1$, $X_2$ et $Y_1$ représentent un atome de carbone ; ou
- $Y_2$ représente un atome d'azote et $X_1$, $X_2$ et $Y_1$ représentent un atome de carbone ; ou
- $X_2$ représente un atome d'oxygène, $Y_2$ représente un atome d'azote et $X_1$ et $Y_1$ représentent un atome de carbone ; ou
- $X_2$ représente un atome de soufre, $Y_2$ représente un atome d'azote et $X_1$ et $Y_1$ représentent un atome de carbone ; ou
- $X_2$ et $Y_2$ représentent un atome d'azote et $X_1$ et $Y_1$ représentent un atome de carbone ; ou
- $Y_1$ représente un atome d'azote et $Y_2$ représente un atome d'oxygène et $X_1$ et $X_2$ représentent un atome de carbone ; ou
- $Y_1$ représente un atome d'oxygène et $Y_2$ représente un atome d'azote et $X_1$ et $X_2$ représentent un atome de carbone ; ou
- $X_2$, $Y_1$ et $Y_2$ représentent un atome d'azote et $X_1$ représente un atome de carbone ; ou
- $X_1$, $X_2$ et $Y_2$ représentent un atome d'azote et $Y_1$ représente un atome de carbone.

Parmi les composés selon la présente invention, on préfère ceux dans lesquels $X_1$ et $Y_2$ représentent un atome d'azote et $X_2$ et $Y_1$ représentent un atome de carbone, ainsi que les composés dans lesquels $X_1$, $X_2$ et $Y_2$ représentent un atome d'azote et $Y_1$ représente un atome de carbone.

Parmi les composés selon la présente invention, on préfère également les composés dans lesquels R est l'atome d'hydrogène ou le groupe $-COCH_3$.

Les composés de formule I selon l'invention peuvent être préparés en mettant en oeuvre les méthodes de glycosylation connues de l'homme de métier, notamment :

a) la méthode de HELFERICH décrite dans l'ouvrage « The Carbohydrate, Chemistry and Biochemistry », 2ème édition, Academic Press, New-York-Londres 1972, Tome IA pages 292-294, par condensation d'un sucre peracétylé avec un hydroxyhétérocycle aromatique en présence d'un acide de Lewis ;
b) la méthode de KOENIGS-KNORR (idem, pages 295-299) par condensation d'un acylose halogéné avec un groupe hydroxy à caractère phénolique en présence d'un accepteur de protons, tel que le cyanure mercurique, l'imidazolate d'argent ou le trifluorométhylsulfonate d'argent ;
c) la méthode de MITSONOBU (Duynstee et al., Tet. Lett. 39 (1998), p.4129-4132) par condensation d'un acylose partiellement acétylé et d'un hydroxyhétérocycle aromatique en présence d'un alkylazodicarboxylate et d'une base de Lewis ;

Les composés de formule I sont préparés de préférence selon des méthodes dérivées des procédés référencés

ci-dessus.

Selon un premier procédé général, on effectue les étapes consistant à :

a) faire réagir un système aromatique comportant un groupe hydroxy à caractère phénolique de formule :

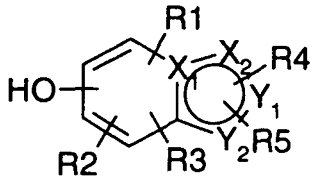

II

dans laquelle :

- $X_1$ et $X_2$ représentent chacun un atome de carbone ou d'azote,
- $Y_1$ et $Y_2$ représentent un atome de carbone, d'azote, de soufre ou d'oxygène, avec la condition que si $Y_2$ représente un atome d'oxygène ou de soufre, $Y_1$ représente un atome de carbone ou d'azote,
- $R_1$, $R_2$, $R_3$, $R_4$ et $R_5$ représentent, indépendamment l'un de l'autre, un atome d'hydrogène, un groupe $-COOR_6$ où $R_6$ représente un atome d'hydrogène ou un alkyle en $C_1$-$C_4$, un groupe alkyle en $C_1$-$C_4$ éventuellement substitué par un noyau phényle, un atome d'halogène ou un groupe $-COOR_6$, un groupe alcoxy en $C_1$-$C_4$, un groupe acyle en $C_2$-$C_6$, un groupe benzoyle ou un noyau phényle,

avec un dérivé du 5-thioxylopyranose de formule :

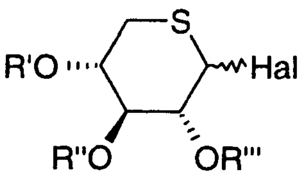

(III-D)

dans laquelle Hal représente un halogène, préférentiellement le brome, et R', R" et R"' représentent un groupe acyle en $C_2$-$C_6$, préférentiellement le groupe acétyle, dans un solvant aprotique tel que l'acétonitrile ou le toluène, en présence d'un sel d'argent, notamment l'oxyde ou l'imidazolate d'argent, ou d'un sel de zinc (notamment l'oxyde ou 1e chlorure) en milieu anhydre, à une température comprise entre 25 et 110°C et pendant 1 à 10 heures, pour obtenir le composé de formule :

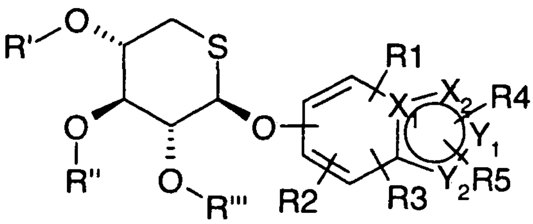

I

dans laquelle $X_1$, $X_2$, $Y_1Y_2$, R', R", R"', $R_1$, $R_2$, $R_3$, $R_4$ et $R_5$ conservent la même signification que dans les composés de départ ;

b) si nécessaire, faire réagir le composé de formule 1 obtenu ci-dessus avec une solution d'ammoniac dans du méthanol pour réaliser la désacylation du reste thioxylopyranosyle et ainsi remplacer les groupes acyles par des atomes d'hydrogène et obtenir le composé de formule :

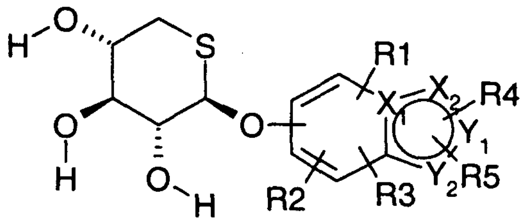

Ia

dans laquelle $X_1$, $X_2$, $Y_1$, $Y_2$, $R_1$, $R_2$, $R_3$, $R_4$ et $R_5$ conservent la même signification que ci-dessus ;

c) si nécessaire, faire réagir l'un des composés I ou Ia obtenus ci-dessus avec un acide selon des méthodes connues de l'homme de métier pour obtenir le sel d'addition correspondant.

En variante de l'étape b) décrite ci-dessus, le remplacement du groupe acyle par un atome d'hydrogène peut être réalisé par action d'un alcoolate métallique, préférentiellement le méthylate de sodium en quantité catalytique, dans le méthanol, à une température comprise entre 0 et 30°C et pendant 0,5 à 2 heures pour obtenir le composté de formule Ia à partir du composé de formule I dans laquelle R représente un groupe acyle en $C_2$-$C_6$.

Selon un second procédé, les composés de formule I peuvent être obtenus par action du tétra-O-acétyl-5-thioxylopyranose de formule :

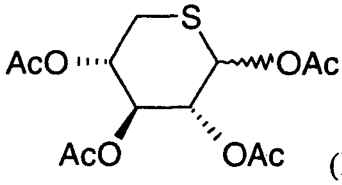

(IV-D)

dans laquelle Ac représente le groupe acétyle
avec un composé de formule :

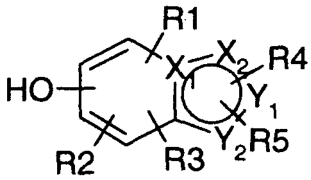

II

dans laquelle :

- $X_1$ et $X_2$ représentent chacun un atome de carbone ou d'azote,
- $Y_1$ et $Y_2$ représentent un atome de carbone, d'azote, de soufre ou d'oxygène, avec la condition que si $Y_2$ représente un atome d'oxygène ou de soufre, $Y_1$ représente un atome de carbone ou d'azote,
- $R_1$, $R_2$, $R_3$, $R_4$ et $R_5$ représentent, indépendamment l'un de l'autre, un atome d'hydrogène, un groupe $-COOR_6$ où $R_6$ représente un atome d'hydrogène ou un alkyle en $C_1$-$C_4$, un groupe alkyle en $C_1$-$C_4$ éventuellement substitué par un noyau phényle, un atome d'halogène ou un groupe $-COOR_6$, un groupe alcoxy en $C_1$-$C_4$, un groupe acyle en $C_2$-$C_6$, un groupe benzoyle ou un noyau phényle,

dans un solvant aprotique, tel que par exemple le dichlorométhane, en présence d'un catalyseur de type acide de Lewis, par exemple le tétrachlorure d'étain, à une température comprise entre 20 et 60°C et pendant 1 à 2 heures, pour obtenir le composé de formule :

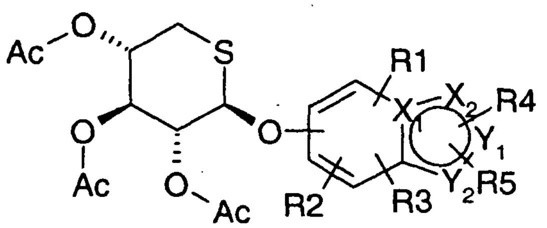

Ib

dans laquelle $X_1$, $X_2$, $Y_1$, $Y_2$, $R_1$, $R_2$, $R_3$, $R_4$ et $R_5$ conservent la même signification que dans les composés de départ.

Le composé de formule Ib peut ensuite être mis en réaction selon le protocole décrit dans le procédé précédent pour obtenir le composé pyranosyle non substitué de formule Ia et/ou un sel avec un acide.

Les composés de formule II précités sont des produits qui peuvent être commerciaux ou facilement synthétisés par l'homme du métier selon des techniques décrites dans la littérature.

Selon un troisième procédé, les composés selon l'invention peuvent encore être préparés par glycosylation directe, selon un procédé consistant à faire réagir un dérivé hétéroaromatique présentant un groupe hydroxy à caractère phénolique avec du 2,3,4-tri-O-acétyl-5-thio-D-xylopyranose, en présence d'un composé alkylazodicarboxylate, comme le diéthylazodicarboxylate, et d'une base de Lewis, comme la triphénylphosphine, d'un solvant polaire aprotique, comme le tétrahydrofurane, à une température comprise entre -20°C et 70°C, pendant 5 minutes à 72 heures, pour obtenir le composé glycosylé correspondant.

Ce composé peut ensuite être mis en réaction selon le protocole décrit dans le procédé précédent pour obtenir le composé pyranosyle non substitué de formule Ia et/ou un sel avec un acide.

Les composés selon l'invention dans lesquels $X_1$ et $Y_2$ représentent un atome d'azote et $X_2$ et $Y_1$ représentent un atome de carbone (dérivés de la famille des imidazo[1,2-*a*]pyridines) de formule :

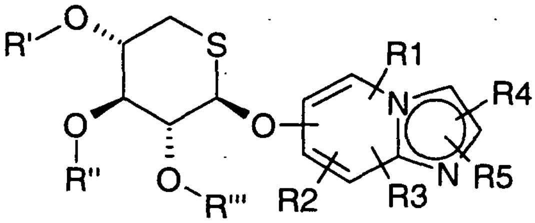

V

peuvent également être préparés par cyclisation entre une pyridine glycosylée présentant un groupe amine en position ortho de l'azote du cycle pyridinique et un dérivé chloré carboxylé (J.J.Kaminski et al, J.Med.Chem, 28(7), 1985 p 876).

Selon ce procédé, on effectue les étapes consistant à :

a) faire réagir un composé de formule :

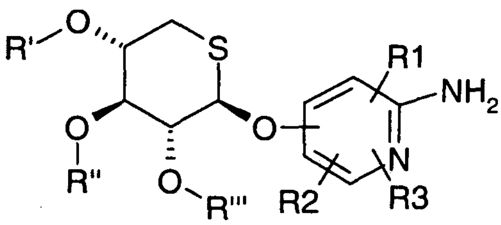

VI

dans laquelle R', R" et R"' représentent un groupe acyle en $C_2$-$C_6$, $R_1$, $R_2$, $R_3$ représentent indépendamment un atome d'hydrogène, un groupe alkyle en $C_1$-$C_4$ éventuellement substitué par un noyau phényle ou un atome d'halogène, un groupe alcoxy en $C_1$-$C_4$, un groupe acyle en $C_2$-$C_6$, un groupe benzoyle ou un noyau phényle,
avec une cétone α-halogénée de formule:

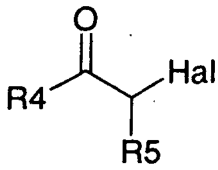

VII

dans laquelle Hal représente un atome d'halogène, de préférence chlore ou brome, et $R_4$ et $R_5$ représentent indépendamment un atome d'hydrogène, un groupe - $COOR_6$ ou $R_6$ représente un atome d'hydrogène ou un alkyle en $C_1$-$C_4$, un groupe alkyle en $C_1$-$C_4$ éventuellement substitué par un noyau phényle, un atome d'halogène ou un groupe $COOR_6$, un groupe alcoxy en $C_1$-$C_4$, un groupe acyle en $C_2$-$C_6$, un groupe benzoyle ou un noyau phényle ; en présence d'un solvant polaire protique, comme l'éthanol, à une température comprise entre 60°C et 130°C, pendant 5 minutes à 4 heures, pour obtenir le composé de formule :

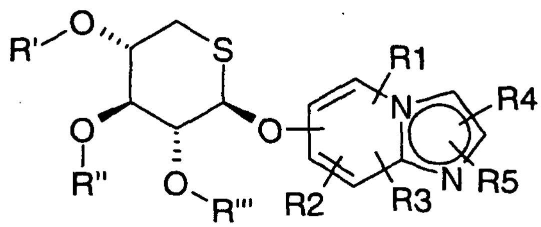

V

dans laquelle R, $R_1$,$R_2$, $R_3$, $R_4$ et $R_5$ conservent la même signification que dans les produits de départ,
b) si nécessaire, effectuer une réaction de déprotection du groupe 2,3,4-triO-acétyl-5-thio-β-D-xylopyranosyle , pour obtenir le composé de formule V dans laquelle R', R" et R"' représentent un atome d'hydrogène.

Les composés selon l'invention dans lesquels $X_1$, $X_2$ et $Y_2$ représentent un atome d'azote et $Y_1$ représente un atome de carbone (dérivés de la famille de la [1,2,4]triazolo[1,5-a]pyridine) de formule :

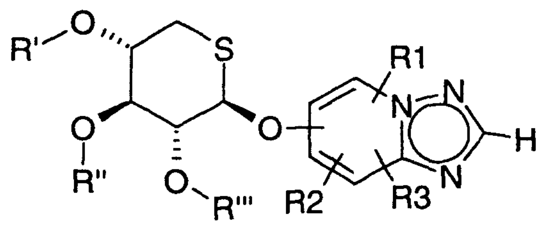

VIII

peuvent également être préparés par cyclisation entre une *N,N*-diméthyl-*N*'-2-pyridinyl-méthanimidamide glycosylée et l'acide hydroxylamine-O-sulfonique.
Selon ce procédé, on effectue les étapes consistant à :

a) faire réagir un composé de formule :

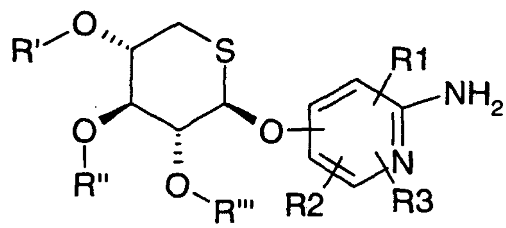

IX

dans laquelle R', R" et R"' représentent un groupe acyle en $C_2$-$C_6$, $R_1$, $R_2$, $R_3$ représentent indépendamment un atome d'hydrogène, un groupe alkyle en $C_1$-$C_4$ éventuellement substitué par un noyau phényle ou un atome d'halogène, un groupe alcoxy en $C_1$-$C_4$, un groupe acyle en $C_2$-$C_6$, un groupe benzoyle ou un noyau phényle, avec le diacétal de la diméthylformamide :

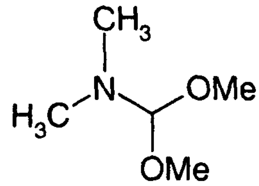

X

en présence d'un solvant polaire protique, comme l'éthanol, à une température comprise entre 60°C et 130°C, pendant 5 minutes à 4 heures, pour obtenir le composé de formule :

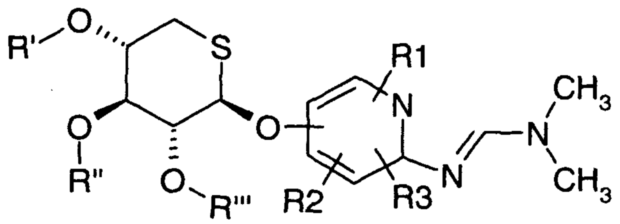

XI

dans laquelle R', R", R"', $R_1$, $R_2$ et $R_3$ conservent la même signification que dans les produits de départ,
b) faire réagir ce composé de formule XI avec l'acide hydroxylamine-O-sulfonique, dans un solvant polaire comme par exemple le méthanol, en présence de pyridine et à une température voisine de la température ambiante pendant 1 à 3 heures, pour obtenir le [1,2,4]triazolo[1,5-a]pyridinyl 2,3,4-tri-O-acétyl-5-thio-β-D-xylopyranoside de formule VIII,
c) si nécessaire, effectuer une réaction de déprotection du groupe 2,3,4-triO-acétyl-5-thio-β-D-xylopyranosyle, pour obtenir le composé de formule VIII dans laquelle R', R" et R"' représentent un atome d'hydrogène.

Selon l'invention, certains composés de formule I sont tels que R' et R" représentent ensemble un pont 1-méthyléthylidène et R"' représente un atome d'hydrogène, ou, R" et R"' représentent ensemble un pont 1-méthyléthylidène et R' représente un atome d'hydrogène. Ces composés peuvent être respectivement représentés par les formules suivantes :

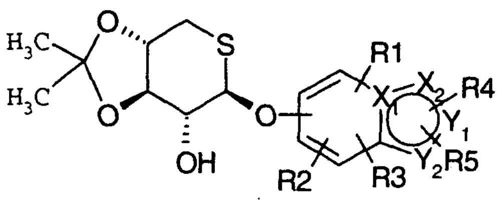

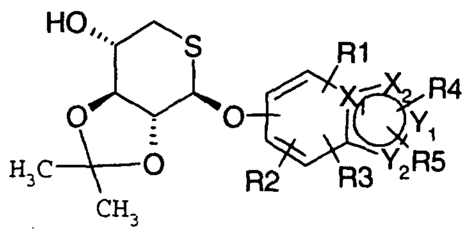

Ces composés peuvent être obtenus au départ d'un composé de formule I dans laquelle R', R" et R"' représentent un atome d'hydrogène, par réaction du 2-méthoxypropène, dans un solvant polaire anhydre tel que par exemple la diméthylformamide, en présence d'un acide tel que l'acide camphresulfonique à une température comprise entre 15 et 70°C et pendant 2 à 48 heures. Les composés sont ensuite isolés et purifiés selon des méthodes connues de l'homme de métier, par exemple par cristallisation ou par chromatographie.
D'une façon générale on préfère utiliser le bromure de 2,3,4-tri-O-acétyl-5-thio-α-D-xylopyranosyle ou le tétra-O-acétyl-5-thio-α-D-xylopyranose quand il s'agit d'obtenir un dérivé du β-D-5-thio-xylopyranose.
Les réactions de glycosylation décrites précédemment conduisent le plus souvent à un mélange des isomères de configuration α et β et il est généralement nécessaire d'optimiser les conditions opératoires afin d'obtenir des proportions favorables à l'isomère de configuration β. Pour cette même raison, il peut être aussi nécessaire d'effectuer des

purifications soit par recristallisation, soit par chromatographie, pour obtenir l'isomère β pur.

Les exemples suivants ont pour but d'illustrer l'invention, et ne sauraient en aucun cas en limiter la portée. Dans ces exemples, les points de fusion ont été mesurés au banc Kofler ou capillaire et les valeurs spectrales de Résonance Magnétique Nucléaire sont caractérisées par le déplacement chimique calculé par rapport au TMS, par le nombre de protons associés au signal et par la forme du signal (s pour singulet, d pour doublet, t pour triplet, q pour quadruplet, m pour multiplet). La fréquence de travail et le solvant utilisé sont indiqués pour chaque composé. La température ambiante est de 20°C ± 4°C.

Les abréviations suivantes ont été utilisées :

| | |
|---|---|
| mM | signifie millimole ($10^{-3}$ mole) |
| $CHCl_3$ | désigne le chloroforme |
| $CH_3OH$ | désigne le méthanol |
| DME | désigne le diméthoxyéthane |
| DMF | désigne la diméthylformamide |
| DMSO | désigne le diméthylsulfoxyde |
| THF | désigne le tétrahydrofurane |
| TFA | désigne l'acide trifluoroacétique. |

__Préparation 1 :__

**2-amino-3-pyridinyl 2,3,4-tri-O-acétyl-5-thio-β-D- xylopyranoside**

A une solution de 14,5 g (35,4 mM) de 2-nitro-3-pyridinyl 2,3,4-tri-O-acétyl-5-thio-β-D-xylopyranoside dans 290 ml de THF, on ajoute 1,45 g de charbon palladié à 10%. Le mélange est agité sous atmosphère d'hydrogène à température ambiante pendant 15 heures puis filtré. Le filtrat est concentré sous pression réduite. On obtient le produit attendu sous forme d'un solide beige avec un rendement de 98%.

F = 145°C.

$[\alpha]_D^{33} = -68°$ (c = 0,44; DMSO).

__Préparation 2 :__

**Acide 5-[(2,3,4-tri-O-acétyl-5-thio-D-xylopyranosyl)oxy]-1*H*-indole-1-carboxylique, 1,1-diméthyléthyl ester**

A une solution de 2,85 g (9,75 mM) de 2,3,4-tri-O-acétyl-5-thio-D-xylopyranose dans 50 ml de THF, on additionne une solution de 1,75 g (7,51 mM) d'ester tertiobutylique de l'acide 5-hydroxy-1*H*-indole-1-carboxylique dans 25 ml de THF, 2,73 g (13,5 mM) de diisopropylazodicarboxylate et 3,54 g (13,5 mM) de triphénylphosphine. Le mélange réactionnel est agité à 45°C pendant 3 heures puis concentré sous pression réduite. Le résidu d'évaporation est dissout dans de l'acétate d'éthyle et la phase organique est lavée avec une solution de soude 1N, puis avec une solution aqueuse concentrée de chlorure d'ammonium. La phase organique est ensuite séchée sur sulfate de magnésium et concentrée sous pression réduite. Le résidu d'évaporation est purifié par chromatographie sur silice greffée $C_{18}$ en éluant à l'aide d'un mélange acétonitrile/eau (7/3 ; v/v). On obtient le produit désiré sous forme d'un solide jaune pâle avec un rendement de 11%.

F = 58-62°C.

__Préparation 3 :__

**2-méthyl-8-hydroxyimidazo[1,2-*a*]pyridine**

A une solution de méthylate de sodium obtenue à partir de 0,2 g (8,7 mM) de sodium et 20 ml de méthanol, on additionne une solution de 1,27 g (5,8 mM) d'acide diméthylcarbamique, 2-méthylimidazo[1,2-*a*]pyridin-7-yl ester dans 15 ml de méthanol. Le mélange réactionnel est porté à reflux pendant 15 heures puis refroidi. Le pH du milieu est ensuite amené à 8,5 à l'aide d'une solution d'acide sulfurique 2N, puis concentré sous pression réduite. Le résidu d'évaporation est purifié par chromatographie sur gel de silice en éluant à l'aide d'un mélange dichlorométhane/méthanol (9/1 ; v/v). On obtient le produit attendu sous forme d'un solide marron avec un rendement de 87%.

F = 154°C.

**Préparation 4 :**

**2-méthyl-6-méthoxyimidazo[1,2-*a*]pyridine**

A une solution de 0,5 g (mM) de 2-amino-5-méthoxypyridine dans 17 ml d'éthanol, on ajoute 0,74 g (8,04 mM) de chloroacétone. Le mélange est chauffé à reflux pendant 20 heures puis concentré sous pression réduite. Le résidu d'évaporation est additionné d'acétate d'éthyle et la phase organique est lavée à l'aide d'une solution aqueuse saturée de bicarbonate de sodium puis à l'aide d'une solution saturée de chlorure de sodium. La phase organique est ensuite séchée sur sulfate de magnésium, filtrée et concentrée sous pression réduite. Le résidu d'évaporation est purifié par chromatographie sur gel de silice greffée $NH_2$ (groupe amino) en éluant à l'aide d'un mélange toluène/alcool isopropylique (99/1 ; v/v). On obtient le produit attendu sous forme d'une huile orange avec un rendement de 54%.
RMN $^1H$ (300MHz ; DMSO) $\delta$ = 8,15 (d, 1H) ; 7,57 (s, 1H) ; 7,34 (d, 1H) ; 16,94 (dd, 1H) ; 3,77 (s, 3H) ; 2,29 (s, 3H).

**Préparation 5 :**

**2-méthyl-6-hydroxyimidazo[1,2-*a*]pyridine**

On chauffe un mélange composé de 1,92 g (11 mM) de 2-méthyl-6-méthoxyimidazo[1,2-*a*]pyridine et de 7,26 g (45 mM) de bromhydrate de pyridinium jusqu'à fusion pendant 8 heures. Le mélange est ensuite refroidi et additionné d'eau. La phase aqueuse est amenée à pH basique à l'aide de bicarbonate de sodium et extraite à l'acétate d'éthyle. La phase organique est lavée à l'eau, séchée sur sulfate de magnésium, filtrée et concentrée sous pression réduite. On obtient ainsi le produit désiré sous forme d'un solide beige avec un rendement de 30%.
F = 174 °C.

**Préparation 6 :**

***N,N*-diméthyl-*N'*-[3-[(2,3,4-tri-*O*-acétyl-5-thio-□-D- xylopyranosyl) oxy]- 2-pyridinyl]-méthanimidamide**

A une solution de 1 g (2,6 mM) de 2-amino-3-pyridinyl 2,3,4-tri-O-acétyl-5-thio-β-D-xylopyranoside (Préparation 1) dans 10 ml d'éthanol, on ajoute 0,31 g (2,6 mM) de 1,1-diméthoxy-*N,N*-diméthyl-éthanamine (N,N-diméthylformamide, diméthylacétal). Dans un récipient adapté au micro-ondes, on chauffe le mélange sous agitation à 120°C pendant 20 minutes au four micro-ondes. Le mélange réactionnel est ensuite concentré sous pression réduite. Le résidu obtenu est purifié par chromatographie sur gel de silice en éluant à l'aide d'acétate d'éthyle. On obtient le produit désiré sous forme d'un solide blanc avec un rendement de 46%.
F = 120°C.

$[\alpha]_D^{32} = -82°$ (c = 0,19; DMSO).

**Préparation 7**

**2,1-benzisoxazol-7-ol**

A une solution de 1,38 g (9,25 mM) de 7-méthoxy-2,1-benzisoxazole dans 50 ml de dichlorométhane refroidie à -78°C, on ajoute 18,5 ml d'une solution 1M de tribromure de bore dans le dichlorométhane, puis on agite à température ambiante pendant une nuit. Le milieu réactionnel est versé dans l'eau et le mélange est amené à pH = 7-8 par addition d'une solution de carbonate de sodium puis extrait au dichlorométhane. La phase organique est séchée sur sulfate de magnésium et concentrée sous vide. Le produit brut obtenu est purifié par chromatographie sur gel de silice en éluant à l'aide d'un mélange méthylcyclohexane/acétate d'éthyle (gradient de 100/0 à 50/50 ; v/v). Le produit attendu est obtenu sous la forme d'un solide jaune avec un rendement de 10%.
RMN $^1H$ (300MHz ; DMSO) $\delta$= 10,46 (s, 1H) ; 9,69 (s, 1H) 7,11. (d, 1H) ; 6,87 (dd, 1H) ; 6,54 (d, 1H).

**Préparation 8**

**3-méthyl-1,2-benzisoxazol-6-ol**

On porte au reflux durant 4 jours 1,98 g de 1-(2,4-dihydroxyphényl)-éthanone, oxime (12 mM) et 1g de potasse (18 mM) dans un mélange méthanol/eau (100 ml/100ml). Le milieu réactionnel est ensuite concentré sous pression réduite, acidifié avec HCl 1N jusqu'à pH = 1, puis extrait à l'acétate d'éthyle. La phase organique obtenue est séchée

sur sulfate de magnésium et concentrée sous pression réduite. Le résidu d'évaporation est purifié par chromatographie sur gel de silice (gradient de méthylcyclohexane/acétate d'éthyle 70/30 à 30/70; v/v). On obtient le produit attendu sous forme d'un solide blanc (0,39g) avec un rendement de 22%).
F = 122-136°C.

**Exemple 1 :**

**2-méthylimidazo[1,2-*a*]pyridin-8-yl 2,3,4-tri-O-acétyl-5-thio-β-D- xylopyranoside**

Procédé A

A une solution de 3 g (7,8 mM) de 2-amino-3-pyridinyl 2,3,4-tri-O-acétyl-5-thio-β-D-xylopyranoside obtenu selon la préparation 1, dans 15 ml d'éthanol, on ajoute 3,6 g (0,39 mM) de chloroacétone. Dans un récipient adapté au micro-ondes, on chauffe le mélange sous agitation à 120°C pendant 30 minutes. Le mélange réactionnel est ensuite concentré sous pression réduite. Le résidu obtenu est dissout dans l'acétate d'éthyle et la phase organique est lavée successivement à l'aide d'une solution aqueuse saturée de bicarbonate de sodium, d'eau, d'une solution saturée de chlorure de sodium saturée et séchée sur sulfate de magnésium. La phase organique est ensuite filtrée et concentrée sous pression réduite. On obtient 3,8 g de produit brut qui est directement utilisé sans purification supplémentaire pour l'étape suivante (décrite dans l'exemple 2).

Procédé B:

A une solution de 1,32 g (4,5 mM) de 2,3,4-tri-O-acétyl-5-thio-β-D-xylopyranose dans 30 ml de THF refroidie à -10°C, on ajoute 0,44 g (3 mM) de 8-hydroxy-2-méthyl-imidazo[1,2-*a*]pyridine, 1,18 g (4,5 mM) de triphénylphosphine, 3,46 g (4,5 mM) de diéthylazodicarboxylate supporté sur résine polystyrènique (1,3 mM/g). Le mélange réactionnel est agité à -10°C pendant 30 minutes, puis 15 heures à température ambiante. Le mélange réactionnel est ensuite filtré puis concentré sous pression réduite. Le résidu obtenu est dissout dans de l'acétate d'éthyle et la phase organique est lavée avec une solution de soude 1N, puis avec de l'eau. La phase organique est ensuite séchée sur sulfate de magnésium et concentrée sous pression réduite. Le résidu d'évaporation est purifié par chromatographie phase inverse sur silice greffée $C_{18}$ en éluant à l'aide d'un gradient eau/acétonitrile. On obtient le produit désiré sous forme d'un solide blanc avec un rendement de 14%.
F = 180°C.

$[\alpha]_D^{28} = -80°$ (c = 0,25; DMSO).

**Exemple 2 :**

**2-méthylimidazo[1,2-*a*]pyridin-8-yl 5-thio-β-D- xylopyranoside**

On agite à température ambiante pendant 15 heures le produit obtenu selon l'exemple 1, dans 40 ml d'une solution d'ammoniac 7 M dans le méthanol. Le mélange réactionnel est concentré sous pression réduite et le produit brut obtenu est purifié par chromatographie sur gel de silice en éluant à l'aide d'un mélange dichlorométhane/méthanol (gradient de 100/0 à 80/20 ; v/v). Le produit obtenu est agité dans 200 ml d'eau froide puis filtré. On obtient le produit désiré sous forme d'une poudre blanche avec un rendement de 53%.
F = 145°C.

$[\alpha]_D^{30} = -89°$ (c = 0,40; DMSO).

**Exemple 3 :**

**2-phénylimidazo[1,2-*a*]pyridin-8-yl 2,3,4-tri-O-acétyl-5-thio-β-D- xylopyranoside**

En opérant de façon analogue au procédé A de l'exemple 1, au départ de 2-chloro-1-phényléthanone, on obtient le produit désiré sous forme d'un solide écru avec un rendement de 52%.
F = 205°C.

$[\alpha]_D^{29} = -94°$ (c = 0,38; DMSO).

**Exemple 4 :**

**2-phénylimidazo[1,2-*a*]pyridin-8-yl 5-thio-β-D- xylopyranoside**

En opérant de façon analogue à l'exemple 2, au départ du produit obtenu à l'exemple 3, on obtient le produit désiré sous forme d'un solide blanc avec un rendement de 42%.
F = 173°C.

$[\alpha]_D^{29} = -77°$ (c = 0,48; DMSO).

**Exemple 5 :**

**3-méthylimidazo[1,2-*a*]pyridin-8-yl 2,3,4-tri-O-acétyl-5-thio-β-D- xylopyranoside**

En opérant de façon analogue au procédé A de l'exemple 1, au départ de 2-chloropropanal, on obtient le produit désiré sous forme d'un solide blanc avec un rendement de 24%.
F = 150°C.

$[\alpha]_D^{29} = -83°$ (c = 0,25; DMSO).

**Exemple 6 :**

**3-méthylimidazo[1,2-*a*]pyridin-8-yl 5-thio-β-D- xylopyranoside**

En opérant de façon analogue à l'exemple 2, au départ du produit obtenu à l'exemple 5, on obtient le produit désiré sous forme d'un solide blanc avec un rendement de 57%.
F = 119°C.

$[\alpha]_D^{29} = -69°$ (c = 0,38; DMSO).

**Exemple 7**

**2-acétyl-7-benzofuranyl 2,3,4-tri-O-acétyl-5-thio-β-D- xylopyranoside**

On agite un mélange composé de 1,93 g (14,1 mM) de chlorure de zinc anhydre, 1 g (5,7 mM) de 2-acétyl-7-hydroxybenzofurane et 2,2 g de tamis moléculaire 13X, dans 13 ml de toluène et 13 ml d'acétonitrile. Le mélange est porté à 90°C et on ajoute, en maintenant à température, 1,42 g (14,1 mM) de triéthylamine et 2,22 g (6,27 mM) de bromure de 2,3,4-tri-O-acétyl-5-thio-D-xylopyranosyle. Le mélange maintenu à 90°C est encore agité 20 minutes. On refroidit ensuite le mélange, on ajoute 65 ml d'une solution de soude 0,5 N et on agite une heure. Le milieu est filtré et le précipité lavé à l'acétate d'éthyle. Le filtrat est ensuite décanté. La phase organique est lavée par une solution aqueuse saturée de chlorure d'ammonium, séchée sur sulfate de magnésium et concentrée sous pression réduite. Le résidu d'évaporation est purifié par chromatographie sur gel de silice en éluant à l'aide d'un mélange cyclohexane/acétate d'éthyle (7/3 ; v/v). Le produit désiré est obtenu sous la forme d'un solide jaunâtre avec un rendement de 37%.
F = 208-209°C.

$[\alpha]_D^{27} = -77°$ (c = 0,48; DMSO).

**Exemple 8 :**

**2-acétyl-7-benzofuranyl 5-thio-β-D- xylopyranoside**

A une suspension de 1,85 g (4,1 mM) de produit obtenu selon l'exemple 7 dans 40 ml de méthanol, on ajoute, à température ambiante, 0,118 ml d'une solution de méthylate de sodium à 18,7 % en poids dans le méthanol (0,41 mM). Le mélange réactionnel est agité à 40°C pendant 70 minutes. On refroidit ensuite le milieu en agitant puis on ajoute de la résine Amberlite IR 120 H+. La résine est ensuite éliminée par filtration et rincée par un mélange méthanol/tétrahydrofurane. Le filtrat est concentré sous pression réduite et le résidu obtenu est trituré dans l'éther et filtré. Après filtration, le solide obtenu est séché. On obtient ainsi le composé attendu sous la forme de cristaux jaunes avec un rendement de 76%.

F = 116-120°C.

$[\alpha]_D^{24} = -73°$ (c = 0,55; DMSO).

**Exemple 9 :**

**1*H*-indol-5-yl 2,3,4-tri-O-acétyl-5-thio-β-D- xylopyranoside**

A une solution de 0,42 g (0,82 mM) de produit obtenu selon la préparation 2 dans 60 ml de dichlorométhane, on additionne 0,089 g (0,82 mM) d'anisole et 7 ml d'acide trifluoroacétique. Le mélange réactionnel est agité à reflux pendant 1 heure et 30 minutes puis, après refroidissement, concentré sous pression réduite. Le résidu d'évaporation est purifié par chromatographie sur gel de silice en éluant à l'aide d'un mélange toluène/acétone (95/5 ; v/v). On obtient le produit désiré sous forme d'une huile incolore avec un rendement de 30%.

**Exemple 10 :**

**1*H*-indol-5-yl 5-thio-β-D- xylopyranoside**

En opérant de façon analogue à l'exemple 8, au départ du produit obtenu à l'exemple 9, on obtient le produit désiré sous forme d'un solide blanc avec un rendement de 53%.
RMN (250 MHz, DMSO) δ= 10,95 (s, 1H ), 7,29 (m, 3H), 6,84 (dd, 1H), 6,35 (m, 1H), 5,45 (d, 1 H), 5,04 (d, 1H), 4,99 (d, 1 H J= 8,88 Hz), 4,94 (d, 1H), 3,52 (m, 2H), 3,13 (m, 1H), 2,55 (m, 2H).

**Exemple 11 :**

**2-méthyl-5-benzothiazolyl 2,3,4-tri-O-acétyl-5-thio-β-D- xylopyranoside**

En opérant de façon analogue à l'exemple 7, au départ de bromure de 2,3,4-tri-O-acétyl-5-thio-D-xylopyranosyle et de 2-méthyl-5-hydroxybenzothiazole, on obtient le produit désiré sous forme de cristaux blancs avec un rendement de 6%.
F = 158°C (recristallisé dans l'alcool isopropylique).

$[\alpha]_D^{27} = -23°$ (c = 0,48; $CH_3OH$).

**Exemple 12 :**

**2-méthyl-5-benzothiazolyl 5-thio-β-D- xylopyranoside**

En opérant de façon analogue à l'exemple 8, au départ du produit obtenu à l'exemple 11, on obtient le produit désiré sous forme de cristaux blancs avec un rendement de 73%.
F = 210-211°C.

$[\alpha]_D^{27} = -79°$ (c = 0,41; $CH_3OH$).

**Exemple 13 :**

**5-acétyl-4,7-diméthoxy-6-benzofuranyl 2,3,4-tri-O-acétyl-5-thio-β-D- xylopyranoside**

En opérant de façon analogue à l'exemple 7, au départ de bromure de 2,3,4-tri-O-acétyl-5-thio-D-xylopyranosyle et de 5-acétyl-4,7-diméthoxy-6-hydroxybenzofurane, on obtient le produit désiré qui est directement engagé dans l'étape suivante de déprotection sans purification préalable.

**Exemple 14 :**

**5-acétyl-4,7-diméthoxy-6-benzofuranyl 5-thio-β-D- xylopyran oside**

En opérant de façon analogue à l'exemple 2, au départ du produit obtenu à l'exemple 13, on obtient le produit désiré sous forme d'un solide blanc avec un rendement de 25%.

F = 207-209°C.

$[\alpha]_D^{26} = -40°$ (c = 0,29; DMSO).

**Exemple 15 :**

**4-benzoxazolyl 2,3,4-tri-O-acétyl-5-thio-β-D- xylopyranoside**

En opérant de façon analogue à l'exemple 7, au départ de bromure de 2,3,4-tri-O-acétyl-5-thio-D-xylopyranosyle et de 4-hydroxybenzoxazole, on obtient le produit désiré sous forme d'un solide blanc avec un rendement de 41%.
F = 139°C.

$[\alpha]_D^{29} = -61°$ (c = 0,23; DMSO).

**Exemple 16**

**4-benzoxazolyl 5-thio-β-D- xylopyranoside**

En opérant de façon analogue à l'exemple 2, au départ du produit obtenu à l'exemple 15, on obtient le produit désiré sous forme d'une poudre blanc cassé avec un rendement de 61 %.
F = 172°C.

$[\alpha]_D^{29} = -94°$ (c = 0,19; DMSO).

**Exemple 17 :**

**7-benzofuranyl 2,3,4-tri-O-acétyl-5-thio-β-D- xylopyranoside**

En opérant de façon analogue à l'exemple 7, au départ de bromure de 2,3,4-tri-O-acétyl-5-thio-D-xylopyranosyle et de 7-hydroxybenzofurane, on obtient le produit désiré sous forme d'un solide blanc avec un rendement de 14%.
F = 152°C:

$[\alpha]_D^{30} = -47°$ (c = 0,32; DMSO).

**Exemple 18 :**

**7-benzofuranyl 5-thio-β-D- xylopyranoside**

En opérant de façon analogue à l'exemple 2, au départ du produit obtenu à l'exemple 17, on obtient le produit désiré sous forme d'un solide blanc avec un rendement de 50%.
F = 160°C.

$[\alpha]_D^{30} = -85°$ (c = 0,24; DMSO).

**Exemple 19 :**

**3-benzoyl-5-benzofuranyl 2,3,4-tri-O-acétyl-5-thio-β-D- xylopyranoside**

En opérant de façon analogue à l'exemple 7, au départ de bromure de 2,3,4-tri-O-acétyl-5-thio-D-xylopyranosyle et de 3-benzoyl-5-hydroxybenzofurane, on obtient le produit désiré sous forme d'un solide blanc avec un rendement de 20%.
F = 182°C.

$[\alpha]_D^{27} = +1°$ (c = 0,22; DMSO).

**Exemple 20 :**

**3-benzoyl-5-benzofuranyl 5-thio-β-D- xylopyranoside**

En opérant de façon analogue à l'exemple 2, au départ du produit obtenu à l'exemple 19, on obtient le produit désiré sous forme d'un solide blanc avec un rendement de 40%.
F = 163°C.

$[\alpha]_D^{30} = -52°$ (c = 0,33; DMSO).

**Exemple 21 :**

**2,3-diméthylimidazo[1,2-*a*]pyridin-8-yl 2,3,4-tri-O-acétyl-5-thio-β-D- xylo**pyranoside

En opérant de façon analogue au procédé B de l'exemple 1, au départ de 8-hydroxy-2,3-diméthylimidazo[1,2-*a*] pyridine, on obtient le produit désiré sous forme d'un solide blanc avec un rendement de 11%.
F = 213°C.

$[\alpha]_D^{28} = -103°$ (c = 0,23; DMSO).

**Exemple 22 :**

**2,3-diméthylimidazo[1,2-*a*]pyridin-8-yl 5-thio-β-D- xylopyranoside**

En opérant de façon analogue à l'exemple 2, au départ du produit obtenu à l'exemple 21, on obtient le produit désiré sous forme d'un solide blanc avec un rendement de 26%.
F = 125-130°C.

$[\alpha]_D^{28} = -99°$ (c = 0,225; DMSO).

**Exemple 23 :**

**Imidazo[1,2-*a*]pyridin-8-yl 2,3,4-tri-O-acétyl-5-thio-β-D- xylopyranoside**

En opérant de façon analogue au procédé A de l'exemple 1, au départ de bromoacétaldéhyde, on obtient le produit désiré sous forme d'un solide écru avec un rendement de 35%.
F = 130°C.

$[\alpha]_D^{28} = -76°$ (c = 0,50; DMSO).

**Exemple 24 :**

**Imidazo[1,2-*a*]pyridin-8-yl 5-thio-β-D- xylopyranoside**

En opérant de façon analogue à l'exemple 2, au départ du produit obtenu à l'exemple 23, on obtient le produit désiré sous forme d'un solide blanc avec un rendement de 53%.
F = 102°C.

$[\alpha]_D^{29} = -52°$ (c = 0,20; DMSO).

**Exemple 25 :**

**2-(1-méthyléthyl)imidazo[1,2-*a*]pyridin-8-yl 2,3,4-tri-O-acétyl-5-thio-β-D- xylopyranoside**

En opérant de façon analogue au procédé A de l'exemple 1, au départ de 2-méthyl-4-bromo-3-butanone, on obtient le produit désiré sous forme d'un solide blanc avec un rendement de 31 %.
F = 182°C.

$[\alpha]_D^{35} = -72°$ (c = 0,20; DMSO).

**Exemple 26 :**

**2-(1-méthyléthyl)imidazo[1,2-*a*]pyridin-8-yl 5-thio-β-D- xylopyranoside**

En opérant de façon analogue à l'exemple 2, au départ du produit obtenu à l'exemple 25, on obtient le produit désiré sous forme d'un solide blanc avec un rendement de 55%.
F = 83°C.

$[\alpha]_D^{35} = -125°$ (c = 0,20; DMSO).

**Exemple 27 :**

**2-méthylimidazo[1,2-*a*]pyridin-7-yl 2,3,4-tri-O-acétyl-5-thio-β-D- xylopyranoside**

En opérant de façon analogue à l'exemple 7, au départ de bromure de 2,3,4-tri-O-acétyl-5-thio-D-xylopyranosyle et de 2-méthyl-7-hydroxyimidazo[1,2-*a*]-pyridine obtenue selon la préparation 3, on obtient le produit désiré sous forme d'un solide blanc avec un rendement de 38%. Le produit obtenu est directement utilisé dans l'étape de désacétylation.

**Exemple 28 :**

**2-méthylimidazo[1,2-*a*]pyridin-7-yl 5-thio-β-D- xylopyranoside**

En opérant de façon analogue à l'exemple 2, au départ du produit obtenu à l'exemple 27, on obtient le produit désiré sous forme d'un solide rosâtre avec un rendement de 35%.
F = 172°C.

$[\alpha]_D^{35} = -107°$ (c = 0,20; DMSO).

**Exemple 29 :**

**1-méthyl-1*H*-benzimidazol-4-yl 2,3,4-tri-O-acétyl-5-thio-β-D- xylopyranoside**

En opérant de façon analogue à l'exemple 7, au départ de bromure de 2,3,4-tri-O-acétyl-5-thio-D-xylopyranosyle et de 4-hydroxy-1-méthyl-1*H*-benzimidazole, on obtient le produit désiré sous forme d'un solide blanc avec un rendement de 38%. Le produit obtenu est directement utilisé dans l'étape de désacétylation.

**Exemple 30 :**

**1-méthyl-1*H*-benzimidazol-4-yl 5-thio-β-D- xylopyranoside**

En opérant de façon analogue à l'exemple 8, au départ du produit obtenu à l'exemple 29, on obtient le produit désiré sous forme d'un solide marron avec un rendement de 70%.
F = 180°C.

$[\alpha]_D^{32} = -87°$ (c = 0,20; DMSO).

**Exemple 31 :**

**2-(chlorométhyl)imidazo[1,2-*a*]pyridin-8-yl 2,3,4-tri-O-acétyl-5-thio-β-D- xylopyranoside**

En opérant de façon analogue au procédé A de l'exemple 1, au départ de 1,3-dichloroacétone, on obtient le produit désiré sous forme d'un solide blanc avec un rendement de 42%.
F = 166°C.

$[\alpha]_D^{29} = -75°$ (c = 0,20; DMSO).

**Exemple 32 :**

**2-(phénylméthyl)imidazo[1,2-*a*]pyridin-8-yl 2,3,4-tri-O-acétyl-5-thio-β-D- xylopyranoside**

A une solution de 0,31 g (0,678 mM) de 2-chlorométhylimidazo[1,2-*a*]pyridin-8-yl 2,3,4-tri-O-acétyl-5-thio-D-xylopyranoside obtenu selon l'exemple 31, dans 4 ml de DME, on ajoute une solution de 0,108 g (1,02 mM) de carbonate de sodium dans 2 ml d'eau, 0,055 g (0,0678 mM) de [1,1'-bis(diphénylphosphino)-ferrocène]-dichloropalladium (II) dichlorométhane et 0,277 g (1,35 mM) de 4,4,5,5-tétraméthyl-2-phényl-1,2,3-dioxaborolane. Le mélange réactionnel est chauffé à l'aide de micro-ondes à 120°C pendant 30 minutes. Après refroidissement, on ajoute de l'eau et on extrait à l'acétate d'éthyle. La phase organique est séchée sur sulfate de sodium et concentrée sous pression réduite. Le résidu d'évaporation est purifié par chromatographie sur gel de silice en éluant à l'aide d'un mélange dichlorométhane/méthanol (97/3 ; v/v) puis cristallisé dans l'éther. On obtient le produit attendu sous forme d'un solide blanc avec un rendement de 42%.
F = 152°C.

$[\alpha]_D^{29} = -83°$ (c = 0,19; DMSO).

**Exemple 33 :**

**2-(phénylméthyl)imidazo[1,2-*a*]pyridin-8-yl 5-thio-β-D- xylopyranoside**

En opérant de façon analogue à l'exemple 2, au départ du produit obtenu à l'exemple 32, on obtient le produit désiré sous forme d'un solide blanc avec un rendement de 90%.
F= 114°C.

$[\alpha]_D^{29} = -81°$ (c = 0,20; DMSO).

**Exemple 34 :**

**2-méthylimidazo[1,2-*a*]pyridin-6-yl 2,3,4-tri-O-acétyl-5-thio-β-D- xylopyranoside**

En opérant de façon analogue à l'exemple 7, au départ de bromure de 2,3,4-tri-O-acétyl-5-thio-D-xylopyranosyle et de 2-méthyl-6-hydroxyimidazo[1,2-*a*]pyridine obtenue selon la préparation 5, on obtient le produit désiré sous forme d'un solide blanc avec un rendement de 13%.
F = 83°C.

$[\alpha]_D^{24} = +35°$ (c = 0,20; DMSO).

**Exemple 35 :**

**2-méthylimidazo[1,2-*a*]pyridin-6-yl 5-thio-β-D- xylopyranoside**

En opérant de façon analogue à l'exemple 8, au départ du produit obtenu à l'exemple 34, on obtient le produit désiré sous forme d'un solide blanc avec un rendement de 66%.
F = 194°C.

$[\alpha]_D^{31} = -21°$ (c = 1,00; DMSO).

**Exemple 36**

**2-méthylimidazo[1,2-*a*]pyridin-8-yl 2,3-*O*-(1-méthyléthylidène)-5-thio-β-D- xylopyranoside**

On prépare une solution de 725 mg (2,66 mM) de 2-méthylimidazo[1,2-*a*]pyridin-8-yl 5-thio-β-D-xylopyranoside (exemple 2) et 406 mg (5,63 mM) de 2-méthoxypropène dans 1,5 ml de DMF à température ambiante puis on la refroidit à -8°C. On ajoute à cette solution 710 mg (3,05 mM) d'acide camphorsulfonique puis on maintient sous agitation à température ambiante pendant 24h. Le milieu réactionnel est versé dans une solution de bicarbonate de sodium (0,5 N) et extrait à l'acétate d'éthyle. La phase organique est lavée avec une solution de chlorure de sodium saturée puis

séchée sur sulfate de magnésium et concentrée sous vide. Le résidu obtenu est purifié par chromatographie sur gel de silice en éluant à l'aide d'un mélange dichlorométhane/méthanol (88/12 ; v/v). On obtient le produit attendu sous forme d'une poudre blanche avec un rendement de 7%.
F = 196°C.

$[\alpha]_D^{30} = -252°$ (c = 0,25; $CHCl_3$).

**Exemple 37 :**

**2-méthylimidazo[1,2-*a*]pyridin-8-yl 3,4-*O*-(1-méthyléthylidène)-5-thio-β-D- xylopyranoside**

Ce composé est obtenu en même temps que le composé de l'exemple 36. Les 2 produits sont séparés lors de la purification sur gel de silice. On obtient le produit attendu sous forme d'une poudre blanche avec un rendement de 16,5%.
F = 190°C.

$[\alpha]_D^{30} = -252°$ (c = 0,2; $CHCl_3$).

**Exemple 38 :**

**[1,2,4]triazolo[1,5-*a*]pyridin-8-yl 2,3,4-tri-O-acétyl-5-thio-β-D- xylopyranoside**

A une solution de 0,22 g (0,5 mM) du composé obtenu selon la préparation 6, dans 5 ml de méthanol, on ajoute 0,1 ml de pyridine et 0,068 g (0,6 mM) d'acide hydroxylamine-O-sulfonique. Le mélange est agité à température ambiante pendant 2h, et on obtient un précipité blanc. Le mélange réactionnel est ensuite concentré sous pression réduite. Le résidu d'évaporation est solubilisé dans du dichlorométhane et la phase organique est lavée à l'eau. La phase organique est ensuite séchée sur sulfate de sodium et concentrée sous pression réduite. Le résidu obtenu est purifié par chromatographie sur gel de silice en éluant à l'aide d'un mélange dichlorométhane/acétate d'éthyle (gradient de 90/10 à 80/20 ; v/v). On obtient le produit désiré (en mélange avec le produit de départ de l'étape précédente) sous forme d'un solide blanc avec un rendement de 30%.
Le produit est ensuite engagé dans l'étape suivante sans purification complémentaire.

**Exemple 39:**

**[1,2,4]triazolo[1,5-a]pyridin-8-yl 5-thio-β-D- xylopyranoside**

On agite à température ambiante pendant 3 heures un mélange constitué du produit obtenu à l'exemple 38, dans 5 ml d'une solution d'ammoniac 7 M dans le méthanol. Le mélange réactionnel est concentré sous pression réduite et le produit brut obtenu est purifié par chromatographie sur gel de silice en éluant à l'aide d'un mélange dichlorométhane/méthanol (97/3 ; v/v). Le produit obtenu est lavé à l'eau froide puis filtré et séché. On obtient le produit désiré sous forme d'un solide blanc avec un rendement de 36%.
F = 155°C.

$[\alpha]_D^{32} = -106°$ (c = 0,2; DMSO).

**Exemple 40 :**

**2,1-benzisoxazol-7-yl 2,3,4-tri-O-acétyl-5-thio-β-D- xylopyranoside**

On agite un mélange composé de 0,25 g (1,85 mM) de chlorure de zinc anhydre, 0,22 g de tamis moléculaire 13X, dans 2 ml de toluène et 2 ml d'acétonitrile et on ajoute 0,1 g (0,74 mM) de 2,1-benzisoxazol-7-ol obtenu selon la préparation 7 et 0,19 g (1,85 mM) de triéthylamine. Le mélange est porté à 90°C et on ajoute, en maintenant à cette température, 0,29 g (0,82 mM) de bromure de 2,3,4-tri-O-acétyl-5-thio-D-xylopyranosyle. Le mélange maintenu à 90°C est encore agité pendant 20 minutes. On refroidit ensuite le mélange, on filtre puis on concentre sous pression réduite. Le résidu d'évaporation est purifié par chromatographie sur gel de silice en éluant à l'aide d'un mélange méthylcyclohexane/acétate d'éthyle (gradient 100/0 à 50/50 ; v/v). Le produit désiré est obtenu sous la forme d'un solide beige avec un rendement de 30%.

F =157°C.

$[\alpha]_D^{25} = -38°$ (c = 0,14; DMSO).

**Exemple 41 :**

**2,1-benzisoxazol-7-yl 5-thio-β-D- xylopyranoside**

On agite à température ambiante pendant 3 heures un mélange constitué du produit obtenu selon l'exemple 40, dans 5 ml de méthanol et 10 gouttes d'une solution de méthylate de sodium à 8% dans le méthanol. Le milieu réactionnel est ensuite neutralisé à l'aide de résine IR 120 $H^+$ à pH = 5, filtré et concentré sous pression réduite. Le produit brut obtenu est purifié par chromatographie sur gel de silice en éluant à l'aide d'un mélange dichlorométhane/méthanol (gradient 100/0 à 80/20 ; v/v) puis par HPLC semi-préparative (colonne : Waters, Atlantis, 19 X 100 mm. Eluant : gradient Acétonitrile/$H_2O$/0,1%TFA). On obtient le produit désiré sous forme d'un solide beige avec un rendement de 32%.
F = 115°C.

$[\alpha]_D^{28} = -116°$ (c = 0,11; DMSO).

**Exemple 42 :**

**Acide 8-[(2,3,4-tri-O-acétyl-5-thio-β-D-xylopyranosyl)oxy]-imidazo[1,2-*a*]-pyridine-2-acétique, éthyl ester**

En opérant de façon analogue au procédé A de l'exemple 1, au départ de 2-amino-3-pyridinyl 2,3,4-tri-O-acétyl-5-thio-β-D-xylopyranoside et de l'ester éthylique de l'acide 4-chloro-3-oxo-butanoique, on obtient le produit désiré sous la forme d'un solide blanc avec un rendement de 54%.
F = 120°C.

$[\alpha]_D^{30} = -75°$ (c = 0,29; DMSO).

**Exemple 43 :**

**Acide 8-[(5-thio-β-D-xylopyranosyl)oxy]-imidazo[1,2-*a*]pyridine-2-acétique, sel avec l'acide trifluoroacétique**

A une solution de 0,55 g (1,11 mM) du composé obtenu selon l'exemple 42 dans 6 ml de THF, on ajoute 6 ml d'eau puis 0,23 g (5,55 mM) de lithine et on agite à température ambiante pendant une nuit. Le mélange réactionnel est concentré sous pression réduite puis acidifié à l'aide d'une solution d'acide chlorhydrique (1 M) à pH = 3-4 et lyophilisé. Le produit obtenu est enfin purifié par HPLC semi-préparative (colonne : Waters, Atlantis, 19 X 100mm. Eluant : gradient Acétonitrile /$H_2O$/0,1%TFA). On obtient le produit désiré sous forme de son sel avec l'acide trifluoroacétique, sous forme d'un coton blanc avec un rendement de 31%.
F = 86°C.

$[\alpha]_D^{27} = -54°$ (c = 0,33; DMSO).

**Exemple 44 :**

**2-méthyl-6-benzothiazolyl 2,3,4-tri-O-acétyl-5-thio-β-D- xylopyranoside**

En opérant de façon analogue à l'exemple 7, au départ de 2-méthyl-6-hydroxybenzothiazole, on obtient le produit désiré sous forme d'un solide écru avec un rendement de 24%.
F=160°C.

$[\alpha]_D^{28} = -3°$ (c = 0,3; DMSO).

**Exemple 45 :**

**2-méthyl-6-benzothiazolyl 5-thio-β-D- xylopyranoside**

En opérant de façon analogue à l'exemple 2, au départ du produit obtenu selon l'exemple 44, on obtient le produit désiré sous forme d'un solide blanc avec un rendement de 70%.
F = 184°C.

$[\alpha]_D^{27} = -40°$ (c = 0,34; DMSO).

**Exemple 46 :**

**2-méthyl-5-benzoxazolyl 2,3,4-tri-O-acétyl-5-thio-β-D- xylopyranoside**

En opérant de façon analogue à l'exemple 7, au départ de 2-méthyl-5 hydroxybenzoxazole, on obtient le produit désiré sous forme d'un solide blanc avec un rendement de 26%.
F = 168°C.

$[\alpha]_D^{28} = -30°$ (c = 0,12; DMSO).

**Exemple 47 :**

**2-méthyl-5-benzoxazolyl 5-thio-β-D- xylopyranoside**

En opérant de façon analogue à l'exemple 2, au départ du produit obtenu selon l'exemple 46, on obtient le produit désiré, après cristallisation dans le 2-propanol sous forme d'un solide blanc avec un rendement de 62%.
F=187-189°C.

$[\alpha]_D^{25} = -86°$ (c = 0,23; DMSO).

**Exemple 48 :**

**2-méthyl-4-benzoxazolyl 2,3,4-tri-O-acétyl-5-thio-β-D- xylopyranoside**

En opérant de façon analogue à l'exemple 7, au départ de 2-méthyl-4-hydroxybenzoxazole, on obtient le produit désiré sous forme d'un solide blanc avec un rendement de 9%.
F = 149-151 °C.

$[\alpha]_D^{25} = -55°$ (c = 0,27; DMSO).

**Exemple 49 :**

**2-méthyl-4-benzoxazolyl 5-thio-β-D- xylopyranoside**

En opérant de façon analogue à l'exemple 2, au départ du produit obtenu selon l'exemple 48, on obtient le produit désiré, après cristallisation dans l'eau, sous forme d'un solide blanc avec un rendement de 91 %.
F = 164-167°C.

$[\alpha]_D^{25} = -73°$ (c = 0,53; DMSO).

**Exemple 50 :**

**3-méthyl-1,2-benzisoxazol-4-yl 2,3,4-tri-O-acétyl-5-thio-β-D- xylopyranoside**

En opérant de façon analogue à l'exemple 7, au départ de 3 méthyl-4-hydroxy-1,2-benzisoxazole, on obtient le produit désiré sous forme d'un solide crème avec un rendement de 14%.
F = 155-158°C (MeOH).

$[\alpha]_D^{25} = -83°$ (c = 0,78; DMSO).

**Exemple 51:**

**3-méthyl-1,2-benzisoxazol-4-yl 5-thio-β-D- xylopyranoside**

En opérant de façon analogue à l'exemple 2, au départ du composé obtenu selon l'exemple 50, on obtient le produit attendu, après cristallisation dans l'eau, sous forme d'un solide blanc avec un rendement de 89%.
F = 165-168°C.

$[\alpha]_D^{28} = -74°$ (c = 0,18; DMSO).

**Exemple 52 :**

**1-méthyl-1*H*-1,2,3-benzotriazol-5-yl 2,3,4-tri-O-acétyl-5-thio-β-D- xylopyranoside**

En opérant de façon analogue à l'exemple 7, au départ du 5-hydroxy-1-méthyl-1*H*-1,2,3-benzotriazole, on obtient le produit désiré sous forme d'un solide blanc avec un rendement de 11 %.
F = 80°C.

$[\alpha]_D^{28} = -9°$ (c = 0,26; DMSO).

**Exemple 53 :**

**1-méthyl-1*H*-1,2,3-benzotriazol-5-yl 5-thio-β-D- xylopyranoside**

En opérant de façon analogue à l'exemple 2, au départ du composé obtenu selon l'exemple 52, on obtient le produit désiré sous forme d'un solide blanc avec un rendement de 61 %.
F = 220°C.

$[\alpha]_D^{25} = -113°$ (c = 0,26; DMSO).

**Exemple 54 :**

**6-benzothiazolyl 2,3,4-tri-O-acétyl-5-thio-β-D- xylo- pyranoside**

En opérant de façon analogue à l'exemple 7, au départ du 6-hydroxybenzothiazole, on obtient le produit attendu sous forme d'un solide blanc avec un rendement de 30%.
F = 166°C.

$[\alpha]_D^{27} = -2,4°$ (c = 0,2; DMSO).

**Exemple 55 :**

**6-benzothiazolyl-5-thio-β-D- xylopyranoside**

En opérant de façon analogue à l'exemple 2, au départ du composé obtenu selon l'exemple 54, on obtient le produit désiré sous forme d'un solide blanc avec un rendement de 72%.
F = 192°C.

$[\alpha]_D^{27} = -48°$ (c = 0,25; DMSO).

**Exemple 56 :**

**Acide 6-[(2,3,4-tri-O-acétyl-5-thio-β-D-xylopyranosyl)oxy]-1*H*-indole-1-carboxylique, 1,1-diméthyléthyl ester**

A une solution de 4,34 g (14,84 mM) de 2,3,4-tri-O-acétyl-5-thio-D-xylopyranose dans 150 ml de THF, on ajoute une solution de 2,66 g (11,41 mM) d'ester tertiobutylique de l'acide 6-hydroxy-1*H*-indole-1-carboxylique dans 50 ml de THF, 4,16 g (20,6 mM) de diisopropylazodicarboxylate et 5,39 g (20,6 mM) de triphénylphosphine. Le mélange réactionnel est agité à 50°C pendant 4 heures puis concentré sous pression réduite. Le résidu d'évaporation est solubilisé dans de l'acétate d'éthyle et la phase organique est lavée avec une solution de soude 1N, puis avec une solution aqueuse concentrée de chlorure d'ammonium. La phase organique est ensuite séchée sur sulfate de magnésium et concentrée sous pression réduite. Le résidu d'évaporation est purifié par chromatographie sur gel de silice en éluant à l'aide d'un mélange méthylcyclohexane/acétate d'éthyle (gradient de 9/1 à 7/3 ; v/v). Le résidu obtenu est ensuite purifié sur chromatographie sur silice greffée C18 en éluant à l'aide d'un mélange acétonitrile/eau (6/4 ; v/v) On obtient le produit désiré sous forme d'un solide blanc avec un rendement de 3%.
F = 147 - 158°C.

$[\alpha]_D^{28} = 14°$ (c = 0,11; DMSO).

**Exemple 57:**

**1*H*-indol-6-yl 2,3,4-tri-O-acétyl-5-thio-β-D- xylopyranoside**

A une solution de 0,043 g (0,085 mM) du composé obtenu selon l'exemple 56 dans 4,5 ml de dichlorométhane, on ajoute 0,092 g (0,085 mM) d'anisole et 0,5 ml d'acide trifluoroacétique. Le mélange réactionnel est agité à reflux pendant 3h puis, après refroidissement, concentré sous pression réduite. Le résidu d'évaporation est purifié par HPLC semi-préparative (colonne : Waters, Atlantis, 19 X 100 mm. Eluant : gradient Acétonitrile /$H_2O$/0,1%TFA). On obtient le produit désiré sous forme d'un solide blanc-gris avec un rendement de 16%.
F = 172-175°C.

$[\alpha]_D^{23} = -10°$ (c = 0,16; DMSO).

**Exemple 58 :**

**3-méthyl-1,2-benzisoxazol-6-yl 2,3,4-tri-O-acétyl-5-thio-β-D- xylopyranoside.**

En opérant de façon analogue à l'exemple 7, au départ du produit obtenu selon la préparation 8, on obtient le produit désiré sous forme d'un solide blanc avec un rendement de 17%.
F = 170-180°C.

$[\alpha]_D^{22} = -7°$ (c = 0,33; DMSO).

**Exemple 59 :**

**3-méthyl-1,2-benzoisoxazol-6-yl 5-thio-β-D- xylopyranoside**

En opérant de façon analogue à l'exemple 41, au départ du composé obtenu selon l'exemple 58, on obtient le produit désiré sous forme d'un solide blanc avec un rendement de 53%.
F = 148-161 °C.

$[\alpha]_D^{23} = -79°$ (c = 0,13; DMSO).

**Exemple 60 :**

**1-méthyl-1*H*-1,2,3-benzotriazol-6-yl 2,3,4-tri-O-acétyl-5-thio-β-D- xylopyranoside**

En opérant de façon analogue à l'exemple 7, au départ du 1-méthyl-1*H*-1,2,3-benzotriazol-6-ol, on obtient le produit désiré sous forme d'un solide blanc avec un rendement de 60%.
F = 143°C.

$[\alpha]_D^{24} = 3°$ (c = 0,2; DMSO).

**Exemple 61 :**

**1-méthyl-1*H*-1,2,3-benzotriazol-6-yl-5-thio-β-D- xylopyranoside**

En opérant de façon analogue à l'exemple 2, au départ du produit obtenu selon l'exemple 60, on obtient le produit désiré, après cristallisation dans l'eau, sous forme d'un solide blanc avec un rendement de 51%.
F=211°C

$[\alpha]_D^{25} = -86°$ (c = 0,24; DMSO).

**Exemple 62 :**

**1,2-diméthyl-1*H*-benzimidazol-4-yl-2,3,4-tri-O-acétyl-5-thio-β-D- xylopyranoside**

En opérant de façon analogue à l'exemple 7, au départ de 1,2 diméthyl-4-hydroxy -1*H*-benzimidazole, on obtient le produit désiré sous forme de poudre beige avec un rendement de 4%.
F = 192°C.

$[\alpha]_D^{24} = -11°$ (c = 0,11 DMSO).

**Exemple 63 :**

**1,2-diméthyl-1*H*-benzimidazol-4-yl-5-thio-β-D- xylopyranoside**

En opérant de façon analogue à l'exemple 2, au départ du produit obtenu selon l'exemple 62, on obtient le produit désiré sous forme d'une poudre brune avec un rendement de 20%.
F = 153°C.

$[\alpha]_D^{26} = -37°$ (c = 0,2; DMSO).

Les structures des composés de formule I décrits ci-dessus sont reprises dans le tableau suivant :

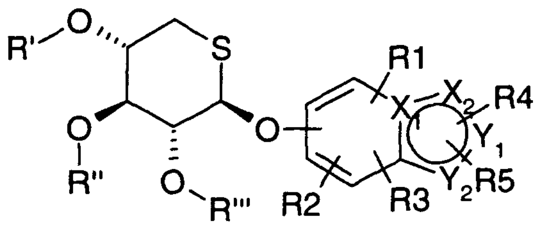

(I)

| Ex | X1 | X2 | Y1 | Y2 | R1 | R2 | R3 | R4 | R5 | R' | R" | R"' | TX |
|---|---|---|---|---|---|---|---|---|---|---|---|---|---|
| 1 | N | C | C | N | H | H | H | 2-$CH_3$ | H | Ac | Ac | Ac | 8 |
| 2 | N | C | C | N | H | H | H | 2-$CH_3$ | H | H | H | H | 8 |
| 3 | N | C | C | N | H | H | H | 2-$C_6H_5$ | H | Ac | Ac | Ac | 8 |
| 4 | N | C | C | N | H | H | H | 2-$C_6H_5$ | H | H | H | H | 8 |
| 5 | N | C | C | N | H | H | H | 3-$CH_3$ | H | Ac | Ac | Ac | 8 |
| 6 | N | C | C | N | H | H | H | 3-$CH_3$ | H | H | H | H | 8 |
| 7 | C | C | C | O | H | H | H | 2-Ac | H | Ac | Ac | Ac | 7 |

(suite)

| Ex | X1 | X2 | Y1 | Y2 | R1 | R2 | R3 | R4 | R5 | R' | R" | R"' | TX |
|---|---|---|---|---|---|---|---|---|---|---|---|---|---|
| 8 | C | C | C | O | H | H | H | 2-Ac | H | H | H | H | 7 |
| 9 | C | C | C | N | H | H | H | H | 2et3-H | Ac | Ac | Ac | 5 |
| 10 | C | C | C | N | H | H | H | H | 2et3-H | H | H | H | 5 |
| 11 | C | S | C | N | H | H | H | 2-$CH_3$ | - | Ac | Ac | Ac | 5 |
| 12 | C | S | C | N | H | H | H | 2-$CH_3$ | - | H | H | H | 5 |
| 13 | C | C | C | O | 4-$OCH_3$ | 7-$OCH_3$ | 5-Ac | H | H | Ac | Ac | Ac | 6 |
| 14 | C | C | C | O | 4-$OCH_3$ | 7-$OCH_3$ | 5-Ac | H | H | H | H | H | 6 |
| 15 | C | N | C | O | H | H | H | H | - | Ac | Ac | Ac | 4 |
| 16 | C | N | C | O | H | H | H | H | - | H | H | H | 4 |
| 17 | C | C | C | O | H | H | H | H | H | Ac | Ac | Ac | 7 |
| 18 | C | C | C | O | H | H | H | H | H | H | H | H | 7 |
| 19 | C | C | C | O | H | H | H | 3-Bz | H | Ac | Ac | Ac | 5 |
| 20 | C | C | C | O | H | H | H | 3-Bz | H | H | H | H | 5 |
| 21 | N | C | C | N | H | H | H | 2-$CH_3$ | 3-$CH_3$ | Ac | Ac | Ac | 8 |
| 22 | N | C | C | N | H | H | H | 2-$CH_3$ | 3-$CH_3$ | H | H | H | 8 |
| 23 | N | C | C | N | H | H | H | H | H | Ac | Ac | Ac | 8 |
| 24 | N | C | C | N | H | H | H | H | H | H | H | H | 8 |
| 25 | N | C | C N | | H | H | H | 2-$CH(CH_3)_2$ | H | Ac | Ac | Ac | 8 |
| 26 | N | C | C | N | H | H | H | 2-$CH(CH_3)_2$ | H | H | H | H | 8 |
| 27 | N | C | C | N | H | H | H | 2-$CH_3$ | H | Ac | Ac | Ac | 7 |
| 28 | N | C | C | N | H | H | H | 2-$CH_3$ | H | H | H | H | 7 |
| 29 | C | N | C | N | H | H | H | 1-$CH_3$ | H | Ac | Ac | Ac | 4 |
| 30 | C | N | C | N | H | H | H | 1-$CH_3$ | H | H | H | H | 4 |
| 31 | N | C | C | N | H | H | H | 2-$CH_2Cl$ | H | Ac | Ac | Ac | 8 |
| 32 | N | C | C | N | H | H | H | 2-Bn | H | Ac | Ac | Ac | 8 |
| 33 | N | C | C | N | H | H | H | 2-Bn | H | H | H | H | 8 |
| 34 | N | C | C | N | H | H | H | 2-$CH_3$ | H | Ac | Ac | Ac | 6 |
| 35 | N | C | C | N | H | H | H | 2-$CH_3$ | H | H | H | H | 6 |
| 36* | N | C | C | N | H | H | H | 2-$CH_3$ | H | H | | 2,3-ip | 8 |
| 37* | N | C | C | N | H | H | H | 2-$CH_3$ | H | 3,4-ip | | H | 8 |
| 38 | N | N | C | N | H | H | H | H | - | Ac | Ac | Ac | 8 |
| 39 | N | N | C | N | H | H | H | H | - | H | H | H | 8 |
| 40 | | C N | O | C | H | H | H | H | - | Ac | Ac | Ac | 7 |

(suite)

| Ex | X1 | X2 | Y1 | Y2 | R1 | R2 | R3 | R4 | R5 | R' | R" | R"' | TX |
|---|---|---|---|---|---|---|---|---|---|---|---|---|---|
| 41 | C | N | O | C | H | H | H | H | - | H | H | H | 7 |
| 42 | N | C | C | N | H | H | H | $-CH_2-CO_2Et$ | H | Ac | Ac | Ac | 8 |
| 43# | N | C | C N | | H | H | H | $-CH_2-CO_2H$ | H | H | H | H | 8 |
| 44 | C | N | C | S | H | H | H | $2-CH_3$ | - | Ac | Ac | Ac | 6 |
| 45 | C | N | C | S | H | H | H | $2-CH_3$ | - | H | H | H | 6 |
| 46 | C | N | C | O | H | H | H | $2-CH_3$ | - | Ac | Ac | Ac | 5 |
| 47 | C | N | C | O | H | H | H | $2-CH_3$ | - | H | H | H | 5 |
| 48 | C | N | C | O | H | H | H | $2-CH_3$ | - | Ac | Ac | Ac | 4 |
| 49 | C | N | C | O | H | H | H | $2-CH_3$ | - | H | H | H | 4 |
| 50 | C | C | N | O | H | H | H | $3-CH_3$ | - | Ac | Ac | Ac | 4 |
| 51 | C | C | N | O | H | H | H | $3-CH_3$ | - | H | H | H | 4 |
| 52 | C | N | N | N | H | H | H | $1-CH_3$ | - | Ac | Ac | Ac | 5 |
| 53 | C | N | N | N | H | H | | H $1-CH_3$ | - | H | H | H | 5 |
| 54 | C | N | C | S | H | H | H | H | - | Ac | Ac | Ac | 6 |
| 55 | C | N | C | S | H | H | H | H | - | H | H | H | 6 |
| 56 | C | C | C | N | H | H | H | $1-CO_2$-tBu | 2et3-H | Ac | Ac | Ac | 6 |
| 57 | C | C | C | N | H | H | H | H | 2et3-H | H | H | H | 6 |
| 58 | C | C | N | O | H | H | H | $3-CH_3$ | - | Ac | Ac | Ac | 6 |
| 59 | C | C | N | O | H | H | H | $3-CH_3$ | - | H | H | H | 6 |
| 60 | C | N | N | N | H | H | H | $1-CH_3$ | - | Ac | Ac | Ac | 6 |
| 61 | C | N | N | N | H | H | | H $1-CH_3$ | - | H | H | H | 6 |
| 62 | C | N | C | N | H | H | H | $1-CH_3$ | $2-CH_3$ | Ac | Ac | Ac | 4 |
| 63 | C | N | C | N | H | H | H | $1-CH_3$ | $2-CH_3$ | H | H | H | 4 |

TX indique la position de substitution du thioxylose
Ac = $COCH_3$
Bz = benzoyl
Bn = benzyle
* : ip signifie un pont isopropylidène (ou 1-méthyléthylidène)
# : sel avec l'acide trifluoroacétique

L'activité antithrombotique des composés selon l'invention a été étudiée *in vivo* chez le rat grâce à un test reproduisant une thrombose veineuse.

La thrombose veineuse a été induite selon le protocole décrit dans Thromb. Haemost. 1992, 67(1), 176-179. L'activité par voie orale a été étudiée selon le protocole opératoire suivant :

L'expérimentation est réalisée sur des rats mâles Wistar, non à jeun, pesant 250 à 280 g et répartis en groupes de 8 à 10 animaux chacun. Les produits à tester sont administrés par voie orale (tubage) en solution ou en suspension dans une solution de méthylcellulose (0,5 % dans l'eau). La concentration des composés est calculée de façon à faire absorber une quantité de solution de 10 ml/kg par voie orale. Une thrombose est induite à un temps T (entre 2 heures et 8 heures) après l'administration du produit et le thrombus formé est prélevé et pesé. Pour induire cette

thrombose, on réalise une stase veineuse sous hypercoagulation, selon la technique décrite par WESSLER (J. Applied Physiol. 1959, 943-946) en utilisant en tant qu'agent hypercoagulant une solution de facteur X activé (Xa), fournie par la société Biogenic (Montpellier), et dosée à 7,5 nKat/kg. La stase veineuse est effectuée 10 secondes exactement après l'injection de l'agent hypercoagulant. L'activité des composés testés a été contrôlée à différentes doses, après qu'ils aient été administrés. L'induction de la thrombose a été faite entre 2 heures et 8 heures après l'administration du composé. A titre d'exemple, les résultats des tests précédents sont reportés dans le tableau suivant pour quelques composés selon l'invention (l'activité est exprimée par le pourcentage d'inhibition de la formation du thrombus, observé en présence du composé selon l'invention, par rapport au poids du thrombus formé en l'absence du composé).

**Tableau I**

| **Activité par voie orale** | | | |
|---|---|---|---|
| Exemple | Dose (mg/kg) | Temps (h) | Activité (%) |
| 2 | 6 | 2 | 94 |
| 8 | 6 | 2 | 91 |
| 12 | 6 | 2 | 74 |
| 16 | 6 | 2 | 80 |
| 18 | 6 | 2 | 62 |
| 39 | 6 | 2 | 100 |
| 43 | 6 | 2 | 53 |

Ces résultats montrent que les composés selon l'invention présentent une activité antithrombotique veineuse.

La présente invention a donc pour objet un composé de formule (I) selon l'invention ainsi que ses sels avec un acide, solvates et hydrates pharmaceutiquement acceptables pour leur utilisation en tant que médicament. Le composé de formule (I) ou un de ses sels, solvates ou hydrates pharmaceutiquement acceptables pourra être utilisé pour la préparation d'un médicament antithrombotique destiné, en particulier, au traitement ou à la prévention des troubles de la circulation veineuse et notamment, pour corriger certains paramètres hématologiques sensibles au niveau veineux.

La présente invention a donc également pour objet des compositions pharmaceutiques contenant un composé de formule (I) ou un de ses sels, solvates ou hydrates pharmaceutiquement acceptables. Ces compositions pharmaceutiques contiennent en général des excipients convenables. Lesdits excipients sont choisis selon la forme pharmaceutique et la voie d'administration souhaitée, en particulier orale ou injectable.

Ces compositions pharmaceutiques sont préparées selon les méthodes classiques bien connues de l'homme du métier. Par exemple, les composés selon l'invention peuvent être formulés avec des excipients physiologiquement acceptables pour obtenir une forme injectable à utiliser directement, une forme injectable à préparer extemporanément ou une forme solide pour administration par voie orale telle que, par exemple, une gélule ou un comprimé.

A titre d'exemple, une forme injectable peut être préparée de préférence par lyophilisation d'une solution filtrée et stérilisée contenant le composé selon l'invention et un excipient soluble en quantité nécessaire et suffisante pour obtenir une solution isotonique après addition extemporanée d'eau pour injection. La solution obtenue pourra être administrée soit en une seule injection sous-cutanée ou intramusculaire, soit sous forme d'une perfusion lente. Une forme administrable par voie orale sera de préférence présentée sous forme d'une gélule contenant le composé de l'invention broyé finement ou mieux, micronisé, et mélangé avec des excipients connus de l'homme du métier, tels que par exemple du lactose, de l'amidon prégélatinisé, du stéarate de magnésium.

Afin d'obtenir l'effet thérapeutique ou prophylactique désiré, chaque dose unitaire peut contenir 10 à 500 mg d'au moins un composé selon l'invention.

**Revendications**

1. Nouveau composé du thioxylose, **caractérisé en ce qu'**il est choisi parmi :

   a) les composés de formule :

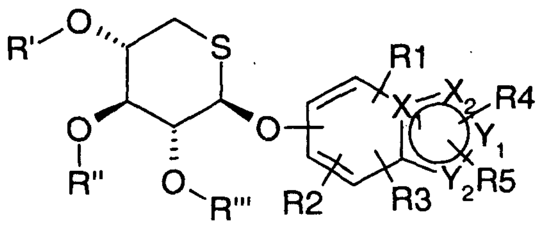

(I)

dans laquelle:

- le groupe pentapyranosyle représente un groupe 5-thio-β-D-xylopyranosyle libre ou substitué,
- R', R" et R"' représentent chacun indépendamment un atome d'hydrogène, un groupe acyle en $C_2$-$C_6$, ou deux contigus d'entre eux forment un pont 1-méthyléthylidène,
- $X_1$ et $X_2$ représentent chacun un atome de carbone ou d'azote,
- $Y_1$ et $Y_2$ représentent chacun indépendamment l'un de l'autre un atome de carbone, d'azote, de soufre ou d'oxygène, avec la condition que si $Y_2$ représente un atome d'oxygène ou de soufre, $Y_1$ représente un atome de carbone ou d'azote,
- $R_1$, $R_2$, $R_3$, $R_4$ et $R_5$ représentent, indépendamment l'un de l'autre, un atome d'hydrogène, un groupe -$COOR_6$ où $R_6$ représente un atome d'hydrogène ou un alkyle en $C_1$-$C_4$, un groupe alkyle en $C_1$-$C_4$ éventuellement substitué par un noyau phényle, un atome d'halogène ou un groupe -$COOR_6$, un groupe alcoxy en $C_1$-$C_4$, un groupe acyle en $C_2$-$C_6$, un groupe benzoyle ou un noyau phényle ;

b) les sels d'addition des composés de formule (I) ;

**2.** Composé selon la revendication 1, **caractérisé en ce que** dans la formule I précitée $R_1$, $R_2$, $R_3$, $R_4$ et $R_5$ représentent, indépendamment l'un de l'autre, un atome d'hydrogène, un groupe alkyle en $C_1$-$C_4$ éventuellement substitué par un noyau phényle ou un atome d'halogène, un groupe alcoxy en $C_1$-$C_4$, un groupe acyle en $C_2$-$C_6$, un groupe benzoyle ou un noyau phényle.

**3.** Composé selon la revendication 1 ou 2, **caractérisé en ce que** dans la formule (I) précitée, $X_1$ et $Y_2$ représentent un atome d'azote et $X_2$ et $Y_1$ représentent un atome de carbone.

**4.** Composé selon l'une des revendications 1 à 3, **caractérisé en ce que** R', R" et R"' dans la formule (I) précitée, représentent un atome d'hydrogène.

**5.** Composé selon l'une des revendications 1 à 3, **caractérisé en ce que** dans la formule (I) précitée, R', R" et R"' représentent un groupe $COCH_3$.

**6.** Procédé de fabrication d'un composé selon l'une quelconque des revendications 1 à 5, **caractérisé en ce qu'**il comprend les étapes consistant à :

a) faire réagir un système aromatique comportant un groupe hydroxy à caractère phénolique de formule :

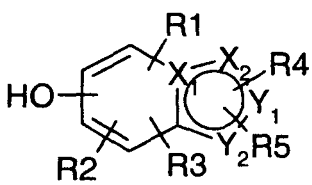

II

dans laquelle :

- $X_1$ et $X_2$ représentent chacun un atome de carbone ou d'azote,
- $Y_1$ et $Y_2$ représentent un atome de carbone, d'azote, de soufre ou d'oxygène, avec la condition que si $Y_2$

représente un atome d'oxygène ou de soufre, $Y_1$ représente un atome de carbone ou d'azote,
- $R_1$, $R_2$, $R_3$, $R_4$ et $R_5$ représentent, indépendamment l'un de l'autre, un atome d'hydrogène, un groupe $-COOR_6$ où $R_6$ représente un atome d'hydrogène ou un alkyle en $C_1$-$C_4$, un groupe alkyle en $C_1$-$C_4$ éventuellement substitué par un noyau phényle, un atome d'halogène ou un groupe $-COOR_6$, un groupe alcoxy en $C_1$-$C_4$, un groupe acyle en $C_2$-$C_6$, un groupe benzoyle ou un noyau phényle,

avec un dérivé du 5-thioxylopyranose de formule :

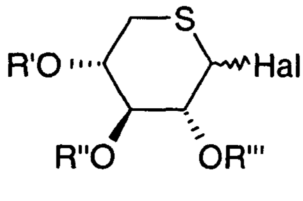

(III-D)

dans laquelle Hal représente un halogène, préférentiellement le brome, et R', R" et R"' représentent un groupe acyle en $C_2$-$C_6$, préférentiellement le groupe acétyle, dans un solvant aprotique tel que l'acétonitrile ou le toluène, en présence d'un sel d'argent, notamment l'oxyde ou l'imidazolate d'argent ou d'un sel de zinc (notamment l'oxyde ou le chlorure), en milieu anhydre, à une température comprise entre 25 et 110°C et pendant 1 à 10 heures, pour obtenir le composé de formule :

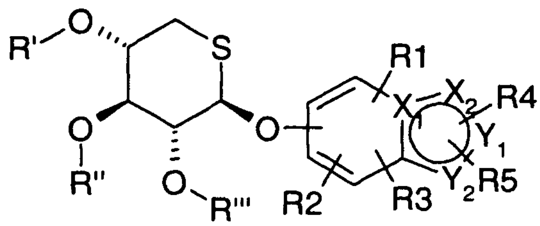

(I)

dans laquelle $X_1$, $X_2$, $Y_1$, $Y_2$, R', R", R"', $R_1$, $R_2$, $R_3$, $R_4$ et $R_5$ conservent la même signification que dans les composés de départ ;

b) si nécessaire, faire réagir le composé de formule I obtenu ci-dessus avec une solution d'ammoniac dans du méthanol pour réaliser la désacylation du reste thioxylopyranosyle et ainsi remplacer les groupes acyles par des atomes d'hydrogène et obtenir le composé de formule :

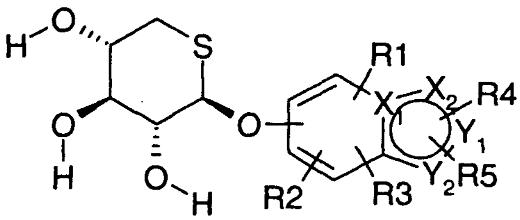

Ia

dans laquelle $X_1$, $X_2$, $Y_1$, $Y_2$, $R_1$, $R_2$, $R_3$, $R_4$ et $R_5$ conservent la même signification que ci-dessus ;

c) si nécessaire, faire réagir l'un des composés I ou Ia obtenus ci-dessus avec un acide selon des méthodes connues de l'homme de métier pour obtenir le sel d'addition correspondant ;

d) si nécessaire, faire réagir le composé de formule Ia obtenu ci-dessus avec le 2-méthoxypropène, dans un solvant anhydre et en milieu acide pour obtenir le composé de formule I dans laquelle deux contigus des substituants R', R" et R"' représentent un pont 1-méthyléthylidène et le troisième représente un atome d'hydrogène.

**7.** Composé selon l'une quelconque des revendications 1 à 5 pour son utilisation en tant que substance pharmacologiquement active.

**8.** Utilisation d'un composé selon l'une quelconque des revendications 1 à 5 pour la préparation d'un médicament destiné à la prévention ou au traitement des thromboses, notamment les thromboses veineuses.

## Claims

**1.** A new thioxylose compound, **characterized in that** it is chosen from:

a) the compounds of formula:

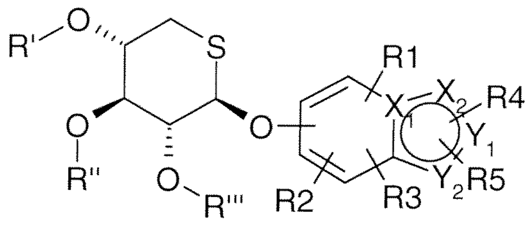

(I)

in which:

- the pentapyranosyl group represents a free or substituted 5-thio-β-D-xylopyranosyl group,
- R', R" and R"' each independently represent a hydrogen atom or a $C_2$-$C_6$ acyl group, or two of them, adjacent, form a 1-methylethylidene bridge,
- $X_1$ and $X_2$ each represent a carbon or nitrogen atom,
- $Y_1$ and $Y_2$ each represent, independently of one another, a carbon, nitrogen, sulfur or oxygen atom, with the condition that, if $Y_2$ represents an oxygen or sulfur atom, $Y_1$ represents a carbon or nitrogen atom,
- $R_1$, $R_2$, $R_3$, $R_4$ and $R_5$ represent, independently of one another, a hydrogen atom, a $-COOR_6$ group where $R_6$ represents a hydrogen atom or a $C_1$-$C_4$ alkyl, a $C_1$-$C_4$ alkyl group optionally substituted with a phenyl ring, a halogen atom or a $-COOR_6$ group, a $C_1$-$C_4$ alkoxy group, a $C_2$-$C_6$ acyl group, a benzoyl group or a phenyl ring;

b) the addition salts of the compounds of formula I;

**2.** A compound according to claim 1, **characterized in that**, in the abovementioned formula I, $R_1$, $R_2$, $R_3$, $R_4$ and $R_5$ represent, independently of one another, a hydrogen atom, a $C_1$-$C_4$ alkyl group optionally substituted with a phenyl ring or a halogen atom, a $C_1$-$C_4$ alkoxy group, a $C_2$-$C_6$ acyl group, a benzoyl group or a phenyl ring.

**3.** A compound according to claim 1 or 2, **characterized in that**, in the abovementioned formula: I, $X_1$ and $Y_2$ represent a nitrogen atom and $X_2$ and $Y_1$ represent a carbon atom.

**4.** A compound according to one of claims 1 to 3, **characterized in that** R', R" and R"' in the abovementioned formula I represent a hydrogen atom.

**5.** A compound according to one of claims 1 to 3, **characterized in that**, in the abovenentioned formula I, R', R" and R"' represent a $COCH_3$ group.

**6.** A process for producing a compound according to any one of claims 1 to 5, **characterized in that** it comprises the steps consisting in:

a) reacting an aromatic system comprising a hydroxyl group that is phenolic in nature, of formula:

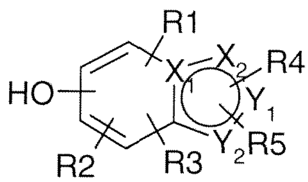

II

in which:

- $X_1$ and $X_2$ each represent a carbon or nitrogen atom,
- $Y_1$ and $Y_2$ represent a carbon, nitrogen, sulfur or oxygen atom, with the condition that, if $Y_2$ represents an oxygen or sulfur atom, $Y_1$ represents a carbon or nitrogen atom,
- $R_1$, $R_2$, $R_3$, $R_4$ and $R_5$ represent, independently of one another, a hydrogen atom, a $-COOR_6$ group where $R_6$ represents a hydrogen atom or a $C_1$-$C_4$ alkyl, a $C_1$-$C_4$ alkyl group optionally substituted with a phenyl ring, a halogen atom or a $-COOR_6$ group, a $C_1$-$C_4$ alkoxy group, a $C_2$-$C_6$ acyl group, a benzoyl group or a phenyl ring,

with a 5-thioxylopyranose derivative of formula:

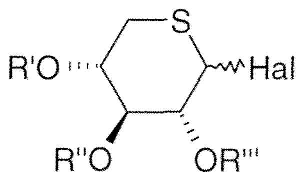

(III-D)

in which Hal represents a halogen, preferably bromine, and R', R'' and R''' represent a $C_2$-$C_6$ acyl group, preferably the acetyl group, in an aprotic solvent such as acetonitrile or toluène, in the presence of a silver salt, in particular silver oxide or silver imidazolate, or of a zinc salt (in particular the oxide or the chloride) in an anhydrous medium, at a temperature of between 25 and 1 !0°C for 1 to 10 hours, so as to obtain the compound of formula:

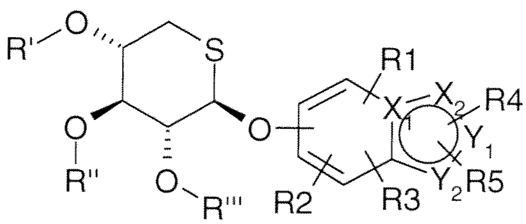

(I)

in which $X_1$, $X_2$, $Y_1$, $Y_2$, R', R'', R''', $R_1$, $R_2$, $R_3$, $R_4$ and $R_5$ keep the same meaning as in the starting compounds;
b) if necessary, reacting the compound of formula I obtained above with a solution of ammonia in methanol so as to perform the deacylation of the thioxylopyranosyl residue and thus to replace the acyl groups with hydrogen atoms and to obtain the compound of formula:

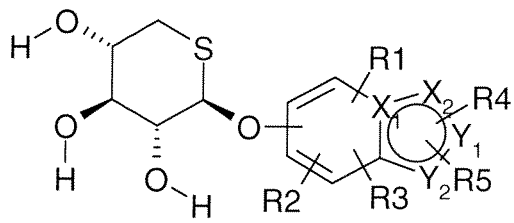

Ia

in which $X_1$, $X_2$, $Y_1$, $Y_2$, $R_1$, $R_2$, $R_3$, $R_4$ and $R_5$ keep the same meaning as above;
c) if necessary, reacting one of the compounds I or Ia obtained above with an acid according to methods known to those skilled in the art, so as to obtain the corresponding addition salt;
d) if necessary, reacting the compound of formula Ia obtained above with 2-methoxypropene, in an anhydrous solvent and in an acidic medium, so as to obtain the compound of formula I in which two of the substituents R', R" and R"', adjacent, represent a 1-methylethylidene bridge and the third represents a hydrogen atom.

**7.** A compound according to any one of claims 1 to 5, for its use as a pharmacologically active substance.

**8.** The use of a compound according to any one of claims 1 to 5, for the preparation of a medicament for use in the prevention or treatment of thromboses, in particular venous thromboses.

**Patentansprüche**

**1.** Neue Thioxylose-Verbindung, **dadurch gekennzeichnet, daß** sie aus folgendem ausgewählt ist:

a) den Verbindungen der Formel

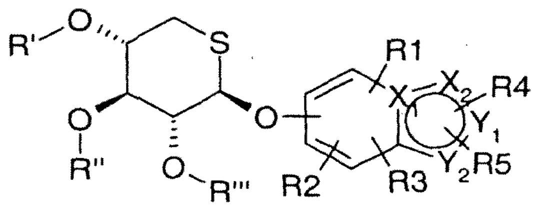

(I)

worin:

- die Pentapyranosyl-Gruppe eine freie oder substituierte 5-Thio-β-D-Xylopyranosyl-Gruppe darstellt,
- R', R" und R"' jeweils unabhängig ein Wasserstoffatom, eine $C_2$-$C_6$-Acylgruppe darstellen oder zwei benachbarte unter ihnen eine 1 -Methylethyliden-Brücke bilden,
- $X_1$ und $X_2$ jeweils ein Kohlenstoff- oder Stickstoffatom darstellen,
- $Y_1$ und $Y_2$ jeweils unabhängig voneinander ein Kohlenstoff-, Stickstoff-, Schwefel- oder Sauerstoffatom darstellen, mit der Bedingung, daß wenn $Y_2$ ein Sauerstoff- oder Schwefelatom darstellt, $Y_1$ ein Kohlenstoff- oder Stickstoffatom darstellt,
- $R_1$, $R_2$, $R_3$, $R_4$ und $R_5$ unabhängig voneinander ein Wasserstoffatom, eine - $COOR_6$-Gruppe, worin $R_6$ ein Wasserstoffatom oder ein $C_1$-$C_4$-Alkyl darstellt, eine $C_1$-$C_4$-Alkylgruppe, eventuell substituiert durch einen Phenylkern, ein Halogenatom oder eine -$COOR_6$-Gruppe, eine $C_1$-$C_4$-Alkoxygruppe, eine $C_2$-$C_6$-Acylgruppe, eine Benzoylgruppe oder einen Phenylkern darstellen,

b) den Additionssalzen der Verbindungen der Formel (I).

**2.** Verbindung nach Anspruch 1, **dadurch gekennzeichnet, daß** in der vorgenannten Formel I $R_1$, $R_2$, $R_3$, $R_4$ und $R_5$ unabhängig voneinander ein Wasserstoffatom, eine $C_1$-$C_4$-Alkylgruppe, eventuell substituiert durch einen Phenylkern oder ein Halogenatom, eine $C_1$-$C_4$-Alkoxygruppe, eine $C_2$-$C_6$-Acylgruppe, eine Benzoylgruppe oder einen Phenylkern darstellen.

**3.** Verbindung nach Anspruch 1 oder 2, **dadurch gekennzeichnet, daß** bei der vorgenannten Formel (I) $X_1$ und $Y_2$ ein Stickstoffatom und $X_2$ und $Y_1$ ein Kohlenstoffatom darstellen.

**4.** Verbindung nach einem der Ansprüche 1 bis 3, **dadurch gekennzeichnet, daß** R', R" und R"' in der vorgenannten Formel (I) ein Wasserstoffatom darstellen.

**5.** Verbindung nach einem der Ansprüche 1 bis 3, **dadurch gekennzeichnet, daß** in der vorgenannten Formel (I) R', R" und R"' eine $COCH_3$-Gruppe darstellen.

**6.** Verfahren zum Herstellen einer Verbindung nach einem der Ansprüche 1 bis 5, **dadurch gekennzeichnet, daß** es die Schritte umfaßt, die darin bestehen:

a) ein aromatisches System, umfassend eine Hydroxygruppe mit phenolischem Charakter, der Formel:

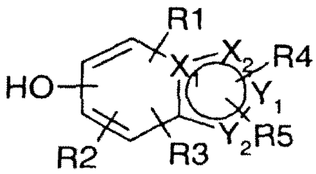

II

worin:

- $X_1$ und $X_2$ jeweils ein Kohlenstoff- oder Stickstoffatom darstellen,
- $Y_1$ und $Y_2$ ein Kohlenstoff-, Stickstoff-, Schwefel- oder Sauerstoffatom darstellen, mit der Bedingung, daß wenn $Y_2$ ein Sauerstoff- oder Schwefelatom darstellt, $Y_1$ ein Kohlenstoff- oder Stickstoffatom darstellt,
- $R_1$, $R_2$, $R_3$, $R_4$ und $R_5$ unabhängig voneinander ein Wasserstoffatom, eine - $COOR_6$-Gruppe, worin $R_6$ ein Wasserstoffatom oder ein $C_1$-$C_4$-Alkyl darstellt, eine $C_1$-$C_4$-Alkylgruppe, eventuell substituiert durch einen Phenylkern, ein Halogenatom oder eine -$COOR_6$-Gruppe, eine $C_1$-$C_4$-Alkoxygruppe, eine $C_2$-$C_6$-Acylgruppe, eine Benzoylgruppe oder einen Phenylkern darstellen,

mit einem 5-Thioxylopyranose-Derivat der Formel:

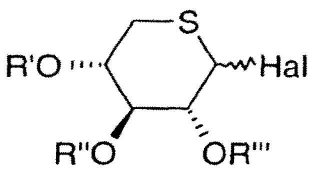

(III-D)

worin Hal ein Halogen, vorzugsweise Brom darstellt und R', R" und R"' eine $C_2$-$C_6$-Acylgruppe, vorzugsweise die Acetylgruppe darstellen, in einem aprotischen Lösungsmittel, wie Acetonitril oder Toluol, in Gegenwart eines Silbersalzes, insbesondere Silberoxid oder -imidazolat, oder eines Zinksalzes (insbesondere dem Oxid oder dem Chlorid), in wasserfreiem Medium, bei einer Temperatur zwischen 25 und 110 °C für 1 bis 10 Stunden reagieren zu lassen, um die Verbindung folgender Formel zu erhalten:

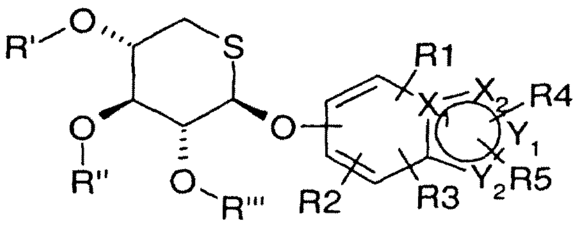

(I)

worin $X_1$, $X_2$, $Y_1$, $Y_2$, R', R", R"', $R_1$, $R_2$, $R_3$, $R_4$ und $R_5$ die gleiche Bedeutung wie bei den Ausgangsverbindungen behalten;

b) falls erforderlich die oben erhaltene Verbindung der Formel I mit einer Lösung aus Ammoniak in Methanol reagieren zu lassen, um die Deacylierung des Thioxylopyranosylrests zu vollziehen und um so die Acylgruppen durch Wasserstoffatome zu ersetzen und die Verbindung folgender Formel zu erhalten:

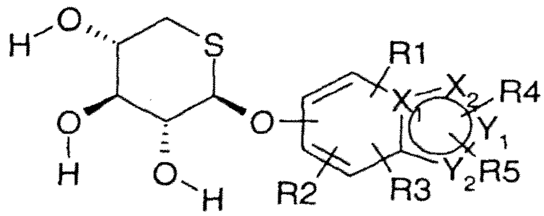

Ia

worin $X_1$, $X_2$, $Y_1$, $Y_2$, $R_1$, $R_2$, $R_3$, $R_4$ und $R_5$ die gleiche Bedeutung wie oben behalten;

c) falls erforderlich der oben erhaltenen Verbindungen I oder Ia nach seitens des Fachmannes bekannten Verfahren mit einer Säure reagieren zu lassen, um das entsprechende Additionssalz zu erhalten;

d) falls erforderlich die oben erhaltene Verbindung der Formel Ia mit 2-Methoxypropen in einem wasserfreien Lösungsmittel und in saurem Medium reagieren zu lassen, um die Verbindung der Formel I zu erhalten, worin zwei benachbarte der Substituenten R', R" und R"" eine 1-Methylethyliden-Brücke darstellen und das dritte ein Wasserstoffatom darstellt.

**7.** Verbindung nach einem der Ansprüche 1 bis 5, für ihre Verwendung als pharmakologisch aktive Substanz.

**8.** Verwendung einer Verbindung nach einem der Ansprüche 1 bis 5, für die Herstellung eines Medikaments, das zur Vorbeugung oder zur Behandlung von Thrombosen, insbesondere von Venenthrombosen bestimmt ist.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 051023 B1
- US 4877808 A
- EP 421829 B1
- WO 05030785 A
- EP 0365397 A
- FR 2860234


### Littérature non-brevet citée dans la description


- *J. Med. Chem.,* vol. 36 (7), 898-903
- *J. Biol. Chem.,* vol. 270 (6), 2662-68
- *Thromb. Haemost.,* 1999, vol. 81, 945-950
- **HELFERICH.** The Carbohydrate, Chemistry and Biochemistry. Academic Press, 1972, 292-294
- **Duynstee et al.** *Tet. Lett.,* 1998, vol. 39, 4129-4132
- **J.J.Kaminski et al.** *J.Med.Chem,* 1985, vol. 28 (7), 876
- *Thromb. Haemost.,* 1992, vol. 67 (1), 176-179
- **WESSLER.** *J. Applied Physiol.,* 1959, 943-946